# EUROPEAN PATENT SPECIFICATION

(11) **EP 0 419 844 B1**
(45) Date of publication and mention of the grant of the patent: **05.04.1995**
(21) Application number: 90116052.3
(22) Date of filing: 22.08.1990
(51) Int. Cl.: C07D 313/12, C07D 495/04, A61K 31/335

(54) **N-hydroxy-dibenz[b,e]oxepin-alkylamines and -alkanoic acid amides, related heterocyclic analogues, a process for their preparation and their use in the manufacture of medicaments**
N-hydroxy-dibenz[b,e]oxepin-alkylamine und -alkanoische Säureamide, verwandte heterocyclische Analoge, Verfahren zu ihrer Herstellung und ihre Verwendung zur Herstellung von Medikamenten
N-hydroxy-dibenz[b,e]oxepin-alkylamines et amides acides alcanoiques, analogues hétérocycliques apparentés, procédé pour leur préparation et leur utilisation pour l'obtention de médicaments

(30) Priority: 25.08.1989 US 398607
(43) Date of publication of application: 03.04.1991
(73) Proprietor: HOECHST-ROUSSEL PHARMACEUTICALS INCORPORATED, Somerville New Jersey 08876 (US)
(72) Inventor: Allen, Richard C., Flemington, NJ 08822 (US); Helsley, Grover Cleveland, Pluckemin, NJ 07978 (US); Hamer, Russell Richard Lee, Lebanon, NJ 08833 (US); Freed, Brian S., Somerset, NJ 08873 (US); White, John I., Harleysville, PA 19438 (US); Martin, Lawrence Leo, Lebanon, NJ 08833 (US)
(74) Representative: Losert, Wolfgang Dr.

(56) References cited:
- EP-A- 0 071 127
- EP-A- 0 119 541
- EP-A- 0 235 796
- DE-A- 2 442 060

## Description

This invention relates to compounds of the formula 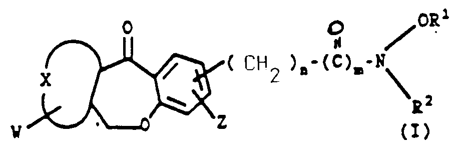
where X together with the carbon atoms to which it is attached forms a benzene or thiophene ring; W and Z are independently hydrogen, halogen, loweralkyl or trifluoromethyl, R¹ is hydrogen, loweralkyl or acyl; R² is, loweralkyl, cycloalkyl, arylloweralkyl or acyl; m is 0 or 1 and n is an integer of 0 to 12 and, where applicable the optical, geometrical and stereoisomers and racemic mixtures.

Preferred embodiments of the inventions are those substituents of Compound I where X together with the carbon atoms to which it is attached forms a benzene or thiophene ring; R¹ is from hydrogen or acyl; and R² is alkyl, cycloalkyl or acyl.

This invention also relates to compounds of the formula 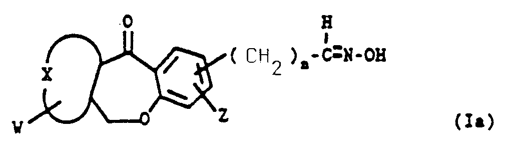
where W, X, Z and n are as previously defined.

This compounds is useful as intermediate for synthesizing Compound I.

From the prior art as represented by DE-A-24 42 060 and EP-A-0 119 541 substituted benzoxepin-amide compounds were known to have anti-inflammatory and analgesic activity.

Throughout the specification and the appended claims, a given chemical formula or name shall encompass all optical, geometrical and stereoisomers thereof and racemic mixtures where such isomers and mixtures exist.

In the above definitions, the term "lower" means the group it is describing contain from 1 to 8 carbon atoms. The term "alkyl" refers to a straight or branched chain hydrocarbon containing no unsaturation, e.g., methyl, ethyl, isopropyl, t-butyl, neopentyl, n-hexyl, etc; the term "cycloalkyl" refers to a monovalent substituent consisting of a saturated hydrocarbon possessing at least one carbocyclic ring of three to eight carbon atoms, e.g. cyclopropyl, cyclobutyl, cyclopentyl, cyclohexyl, cycloheptyl, etc., having its free valence bond from a carbon of the carbocyclic ring; the term "alkoxy" refers to a monovalent substituent which consists of an alkyl group linked through an ether oxygen having its free valence bond from the ether oxygen, e.g. methoxy, ethoxy, propoxy, butoxy, pentoxy, etc., and the term "halogen" refers to a member of the halogen family consisting of fluorine, chlorine, bromine and iodine. The -(CH₂)ₙ- alkylene" refers to a bivalent radical of the lower branched or unbranched alkyl group derived from having valence bonds from two terminal carbons thereof, e.g., methylene (-CH₂-), ethylene (-CH₂CH₂-), propylene (-CH₂CH₂CH₂-), isopropylene 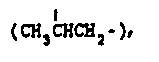
etc.

The compounds of the present invention are prepared in the following manner. The substituents W, X, Z, R¹, R² and R³ and the integers m and n are as defined above unless indicated otherwise.

Compound III of the formula 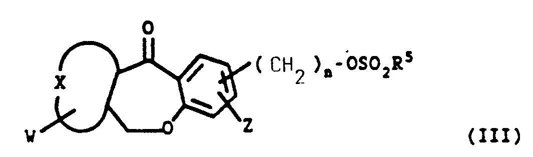
where R⁵ is alkyl, is reacted with an N-alkylhydroxylamine hydrochloride or N-cycloalkylhydroxylamine hydrochloride, e.g., N-methylhydroxylamine hydrochloride, N-ethylhydroxyalamine hydrochloride, N-isopropylhydroxylamine hydrochloride, N-cyclohexylhydroxylamine hydrochloride, etc., in the presence of a strong base, e.g., potassium t-butoxide, sodium methoxide, etc. in a standard amination reaction to form Compound I of the invention, where m=0 and n is not equal to 0; that is, compound IV. 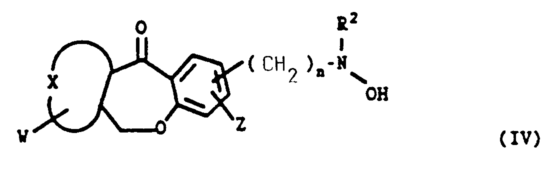
This reaction is carried out under conventional amination reaction conditions, typically in the presence of a polar anhydrous solvent, e.g., methanol, ethanol, propanol or a suitable mixture of such solvents, at a temperature of 0°C to reflux for 1 to 24 hours. Compound III where X is part of a benzene ring, can typically be prepared in the manner described in Martin et al., U.S. Patent No. 4,526,891.

Compound III, where X is part of a thiophene ring, of the formula 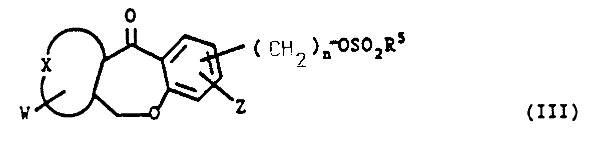
is prepared by the reaction of compound IIIa of the formula 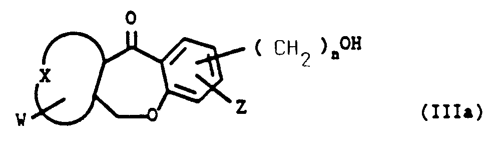
with an alkylsulfonyl halide of the formula

R⁵SO₂Hal

where R⁵ is as previously defined and Hal is halogen. This reaction is carried out under standard conditions, typically in a polar basic solvent, e.g., pyridine, at a temperature of -10°C to 25°C for 1 to 24 hours. Compound IIIa can typically be prepared in the manner described in Martin et al., J. Med. Chem., 27, pp. 372-376 (1984).

The N-alkyl or N-cycloalkyl acid amides of the invention of the formula 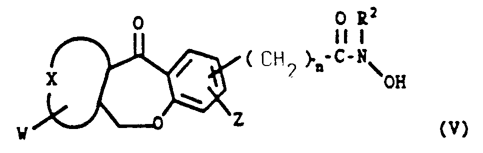
can be prepared in the following manner.

Compound VI of the formula 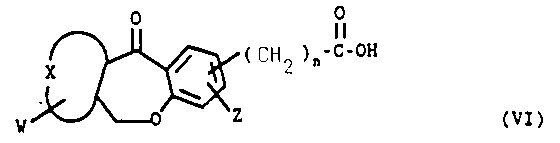
is reacted with a halogenating agent, e.g., thionyl chloride, POCl₃, etc. to afford Compound VII of the formula 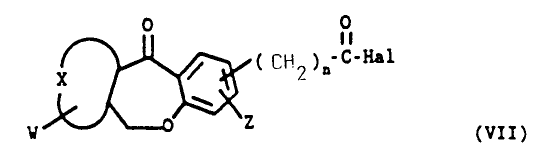
where Hal is halogen. This reaction typically takes place in the presence of a suitable catalyst, e.g., N,N-dimethylformamide and in an inert solvent, e.g., methylene chloride, chloroform, etc. at a temperature of about -10 to 20°C for 1 to 24 hours. Compound VI is typically prepared in the manner described in Martin et al., J. Med. Chem., 27, pp. 372-376 (1984).

Compound VII is reacted with Compound VIII, the salt of an N-alkyl or N-cycloalkylhydroxylamine of the formula

alkyl-NHOH · HCl

or

cycloalkyl-NHOH·HCl (VIII)

where alkyl and cycloalkyl are as previously defined, in the presence of a base, e.g., pyridine, 4-dimethylaminopyridine, etc., to form compound V. This reaction is typically carried out in an ethereal solvent, e.g., tetrahydrofuran, bis(2-methoxyethyl)ether, diethyl ether, etc. at a temperature of from about -10 to 25°C for 1 to 24 hours. Compound VII, where X is part of a benzene ring, is typically prepared in the manner described in Martin et al., U.S. Patent No. 4,515,946.

Compound IX of the formula 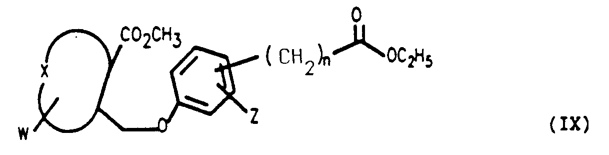
an intermediate for the preparation of compound XI (Compound I where m=1), is prepared by reacting Compound IXa, a (hydroxyphenyl)alkanoic acid ethyl ester of the formula 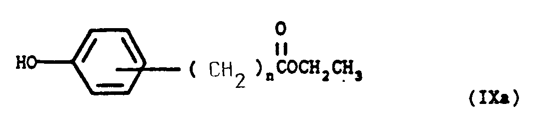
with 2-(halomethyl)benzoic acid methyl ester, compound IXb, of the formula 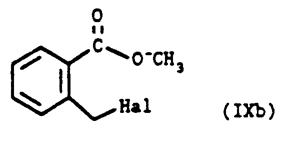
where Hal is halogen. This reaction is typically carried out in the presence of an acid acceptor, e.g., potassium carbonate, sodium carbonate, lithium carbonate, etc., and a catalytic amount of a promoter, i.e., an alkali metal halide, e.g., sodium iodide, potassium iodide, etc. This reaction is carried out under conventional condensation reaction conditions, typically in the presence of a suitable solvent, e.g., 2-butanone, acetone, etc., at a temperature of about 25°C to 80°C to reflux for 1 to 48 hours.

Compound IX is typically cyclized using procedures described in Aultz et al., J. Med. Chem., 20, 1499-1501 (1977) or Martin et al., J. Med. Chem., 27, pp. 372-376 (1984) to form Compound IXc of the formula

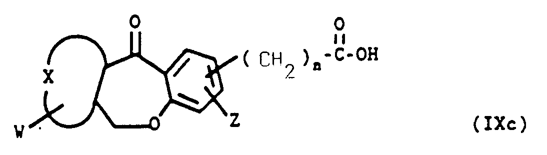

Compound IXc is reacted with a halogenating agent, e.g., thionyl chloride, POCl₃, etc., to afford Compound X of the invention of the formula 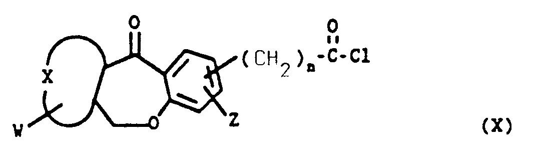
This reaction typically takes place in the presence of a suitable catalyst, e.g., N,N-dimethylformamide, and an inert solvent, e.g., methylene chloride, chloroform, etc., at a temperature of about -10 to 20°C for 1 to 24 hours to reflux.

Compound X in turn is reacted with a solution of an amidating agent, such as the salt of an N-alkyl or N-cycloalkylhydroxylamine of the formula

alkyl-NHOH · HCl

or

cycloalkyl-NHOH · HCl

e.g., N-methylhydroxylamine hydrochloride, N-isopropylhydroxylamine hydrochloride, N-ethylhydroxylamine hydrochlorine and N-cyclohexylhydroxylamine hydrochloride, etc., in an anhydrous basic solvent, such as pyridine, to afford Compound XI of the invention of the formula 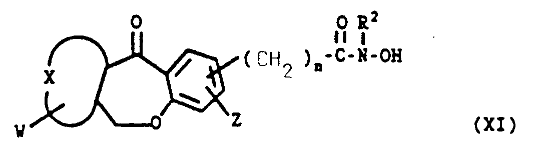
where R² is as previously defined. This reaction is carried out under conventional amidation reaction conditions, typically in the presence of an anhydrous ethereal solvent, e.g., tetrahydrofuran, ether, etc. at a temperature of about 0°C to 25°C for 1 to 24 hours.

Compound I where m=0 can be prepared in the following manner. Compound XII, an intermediate in the synthesis of Compound I, where m=0, 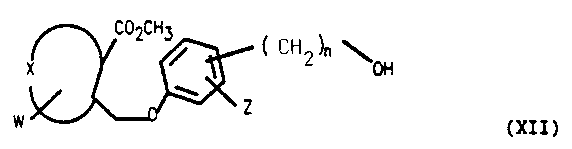
is prepared by reacting, Compound XIIa, an alcohol, a (hydroxyphenyl)alkanol of the formula 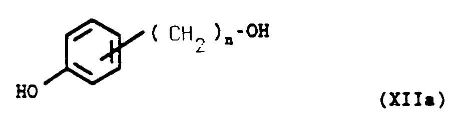
with 2-(halomethyl)benzoic acid methyl ester, (Compound IXb). This reaction is typically carried out in the presence of an acid acceptor, e.g., potassium carbonate, sodium carbonate, lithium carbonate, etc. and a catalytic amount of a promoter, i.e., an alkali metal halide, e.g., potassium iodide, sodium iodide, etc. This reaction is carried out under conventional condensation reaction conditions, typically in the presence of a suitable solvent, e.g., 2-butanone, acetone, etc., at a temperature of about 10°C to 80°C for 1 to 48 hours.

Compound XII is hydrolyzed to afford Compound XIII of formula 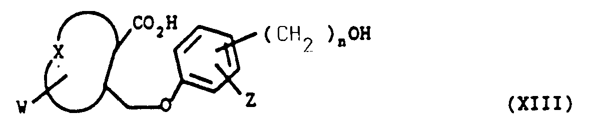
This hydrolysis is typically carried out with a base such as sodium or potassium hydroxide, etc. in a suitable loweralkanol solvent, e.g., methanol, ethanol, propanol, etc. This reaction typically takes place at a temperature of about 25°C to 80°C to reflux for 1 to 24 hours.

Typically, Compound XIII is cyclized by a method that will not interfere with the hydroxy group on the sidechain of the phenyl group, using trifluoroacetic acid anhydride, in an inert solvent, e.g., methylene chloride, as described in Martin et al., U.S. Patent No. 4,496,580, to form Compound XIIIa of the formula

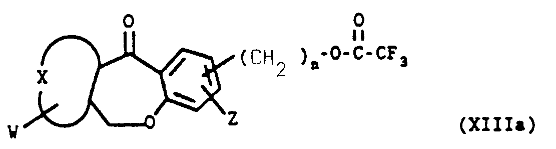

Acetylated Compound XIIIa is hydrolyzed under standard hydrolyzing conditions, e.g., in an aqueous solvent with a mineral acid, e.g., hydrochloric, sulfuric, etc., at a temperature of about 50 to 80°C to reflux for 4 to 24 hours followed by standard basification with a base, e.g., sodium bicarbonate, etc., to form Compound XIV of the invention of the formula

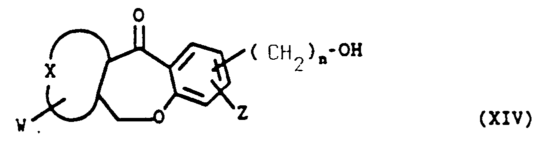

Compound XIV is reacted with an alkylsulfonyl halide in a basic solvent, e.g., pyridine, etc. to form Compound III where n is not equal to 0. Compound III is then reacted as previously described with an N-alkylhydroxylamine hydrochloride or N-cycloalkylhydroxylamine hydrochloride in the presence of a strong base, e.g., potassium t-butoxide, etc. to form Compound IV of the invention, where n is not equal to 0.

Compound XV of the formula 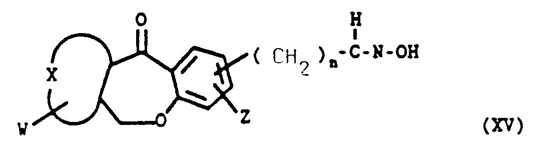
is prepared by the reaction of Compound XVI of the formula 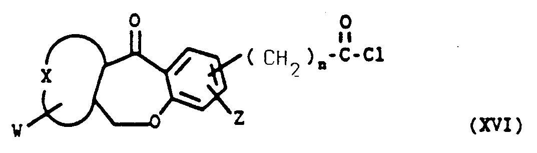
with a reducing agent, e.g., lithium tri-tert-butoxy-aluminohydride in an ethereal solvent, e.g., diglyme, etc., at a temperature of about -80°C to -70°C for 1 to 2 hours to give intermediate XVII of the formula

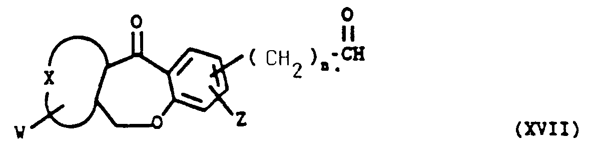

Compound XVII, an aldehyde, is reacted with pyridine and hydroxylamine hydrochloride to afford Compound XV. This condensation reaction typically takes place in an anhydrous basic solvent, such as pyridine at ambient temperature to about 60°C for 1 to 3 hours. Compound XVI is typically prepared in the manner described in Martin et al., U.S. Patent No. 4,515,946.

Compound XVIII of the formula 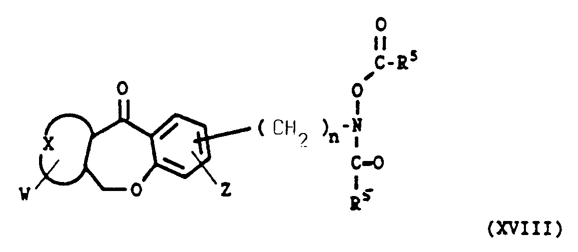
where R⁵ is as previously defined, is prepared in the following manner.

Compound XIX of the formula 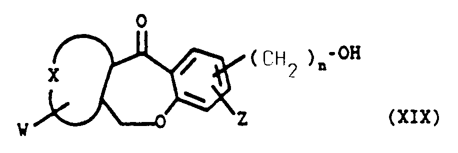
is reacted with an oxidizing agent, e.g., pyridinium chlorochromate in a halogenated solvent, e.g., dichloromethane, etc. under nitrogen, at a temperature of about -20°C to ambient temperature for 1 to 5 hours to form Compound XIXa of the formula 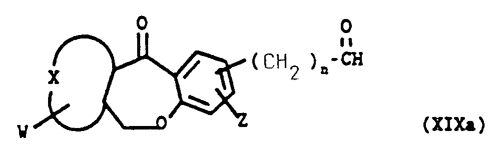
Compound XIXa where n=0 can be prepared from XIX where n=2 via subsequent oxidation of XIXa where n=1 or it can be prepared in the manner described in Yoshioka et al., Journal of Med. Chem, 21, pp. 633-639 (1978).

Compound XVII is reacted with hydroxylamine hydrochloride to form intermediate Compound XX of the formula 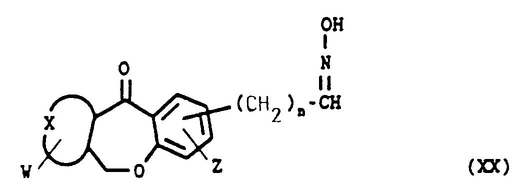
This reaction is carried out under standard conditions, typically in a basic solvent, e.g., pyridine, etc. at ambient to about 60°C for 1 to 5 hours.

Compound XX is reacted with a reducing agent, e.g., boron trihydride, in a polar basic solvent, e.g., pyridine, at a temperature of about 0°C to ambient temperature for 1 to 5 hours to afford Compound XXI of the invention of the formula

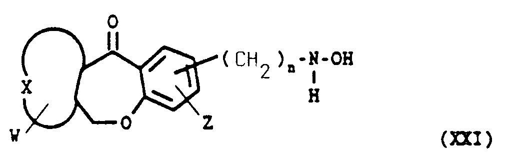

Compound XXI is reacted with a acylating agent, e.g., acetyl chloride, 2-methylpropionyl chloride, etc., in a basic solvent, e.g., pyridine, etc., to form Compound XVIII of the invention. This reaction typically takes place under an inert gas, e.g., nitrogen, at a temperature of about -25°C to ambient temperature for 1/2 to 5 hours.

Compounds of the present invention are useful as topical antiinflamatory agents for the treatment of various dermatoses which may include, for example, exogenous dermatitides (e.g., sunburn, photoallergic dermatitis, urticaria, contact dermatitis, allergic dermatitis), endogenous dermatitis (e.g., atopic dermatitis, seborrheic dermatitis, nummular dermatitis), dermatitides of unknown etiology (e.g., generalized exfoliative dermatitis), and other cutaneous disorders with an inflammatory component (e.g., psoriasis).
The dermatological activities of the compounds were ascertained according to the following methods.

### DERMATOLOGICAL TEST METHODS

### Phospholipase A₂-Induced Paw Edema (PIPE)

The ability of compounds to reverse naja naja (snake venom) phospholipase A₂(PLA₂)-induced paw edema in male Wistar rats (100-125 gm) was measured. PLA₂ (3 units/100 »l dH₂O/paw) alone or with 0.1 M of the test compound was injected in the sulplantar region of the rat left hindpaw. Immediatly following injection and at two hours post administration, the paw was immersed in a mercury bath, and paw displacement was measured on a recorder via a transducer. The value was then converted to mm Hg. (Standards: Quinacrine ED₅₀ = 0.1 M; Hydrocortisone ED₅₀= 0.46 M). Giessler, A.J. et al., "Agents and Action", Vol. 10, "Trends in Inflammation Research" (1981), p. 195.

### Arachidonic Acid-Induced Mouse Ear Edema (AAEE)

In the arachidonic acid-induced ear edema assay, the test compound is dissolved in 30/70 propylene glycol/ethanol and is applied to both ears of groups of 6 female Swiss Webster mice at a volume of 20 »l so that a total of 1.0 mg of test compound as delivered to each ear over the inner and outer surfaces. The mice were housed together in a cage under standard conditions for 1 week prior to use with food and water ad lib. The same volume (20 »l) of vehicle is applied to each ear of a control group of mice. After 30 minutes, arachidonic acid is applied to the right ear of each mouse of each group in the amount of 4 mg/ear. Vehicle is applied to the left ear of each mouse of each group at a volume of 20 »l/ear. After an additional hour, the mice are sacrificed and a 4mm plug is taken from each ear and weighed. The difference between the right and left ear plugs was determined for each animal. The antiinflammatory activity of the test compound is expressed as the mean percent change in the ear plug weight of the treated animals relative to the mean percent change in weights of control animals' ear. (Standard: Indomethacin -90% at 1 mg/ear). Young, J.M. et al., Invest. Dermatol., 80, (1983), pp. 48-52.

### TPA-Induced Ear Edema (TPAEE)

In the TPA-induced ear edema assay, TPA (12-0-tetradecanoyl-phorbol-13-acetate) is dissolved in 30/70 propylene glycol/ethanol and is applied to the right ear of groups of 6 female Swiss Webster mice at a volume of 20 »l so that a total of 10 »g of TPA is delivered to the inner and outer surfaces of the ear. The mice were housed together in a cage under standard conditions for 1 week prior to use with food and water ad lib. The test compound is dissolved in the vehicle and is applied to the right ear (the inner and outer surfaces) at a volume of 20 »l so that a total of 10 »g of the compound is delivered to the ear. After about 5 hours, the animals are sacrificed and a 4 mm diameter plug is taken from each ear and weighed. The difference between the right and left ear plug weights for each animal was determined. The antiinflammatory activity of the test compound is expressed as the mean percent change in ear plug weight of the treated animals compared to the mean percent change in the plug weight of the control animals. (Standard: Indomethacin: -86% at 1 mg/ear). Young, J.M. et al., J. Invest. Dermatol., 80 (1983), pp. 48-52.

Dermatological activities for some of the compounds of this invention are presented in Table 1.

**Table 1**

| Compound | PIPE 0.1 M | AAEE % Change @ 1 mg/ear | TPAEE 10 »g/ear |
|---|---|---|---|
| 2-(6,11-dihydro-11-oxodibenz[b,e]oxepin-2-yl)-N-hydroxy-N-methyl-ethylamine | -70 | -46 | -50 |
| 3-(6,11-dihydro-11-oxodibenz[b,e]oxepin-2-yl)-N-hydroxy-N-methyl-propanamide | -46 | -63 | -71 |
| N-cyclohexyl-2-(6,11-dihydro-11-oxodibenz[b,e]oxepin-2-yl)-N-hydroxyacetamide | -85 | -29 | -52 |

The compounds of the present invention are also useful as analgesic agents due to this ability to alleviate pain in mammals. The activity of the compounds is demonstrated in the phenyl-para-benzoquinone writhing assay in mice, a standard assay for analgesia [Proc. Soc. Exptl. Biol. Med., 95, 729 (1957)].

The analgesic activity of some of the compounds expressed as either the subcutaneous dose at which 50% of the phenyl-para-quinone induced writhing is inhibited in the animals, i.e., the ED₅₀ value, or as the % decrease in writhing at a given dose is presented in Table 2.

**Table 2**

| Compound Writhing | ED₅₀ or % Inhibition of |
|---|---|
| 2-(6,11-Dihydro-11-oxodibenz[b,e]oxepin-2-yl)-N-ethyl-N-hydroxypropanamide | ED₅₀ = 3.6 mg/kg |
| 3-(6,11-Dihydro-11-oxodibenz[b,e]oxepin-2-yl)-N-cyclohexyl-N-hydroxypropanamide | 76% at 20 mg/kg |
| N-Cyclohexyl-2-(6,11-dihydro-11-oxodibenz[b,e]oxepin-2-yl)-N-hydroxyacetamide | 68% at 20 mg/kg |
| Propoxyphene | ED₅₀ = 3.9 mg/kg |

The analgesic relief of pain is achieved when the compounds of the invention are administered to a subject requiring such treatment at an effective oral, parentereal or intravenous dose of from 0.01 to 100 mg/kg of body weight per day. A preferred effective dose within this range is from about 10 to 50 mg/kg of body weight per day. A particularly preferred effective amount is about 30 mg/kg of body weight per day. It is to be understood, however, that for any particular subject, specific dosage regimens should be adjusted according to the individual need and the professional judgment of the person administering or supervising the administration of the compound. It is further to be understood that the dosages set forth herein are exemplary only.

Effective quantities of the compounds of the present invention may be administered to a patient by any of the various methods, for example, orally as in capsules or tablets, topically as in ointments, solutions or salves, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Preferred pharmaceutically acceptable salts of the invention include those derived from inorganic acids such as hydrochloric, hydrobromic, sulfuric, nitric, phosphoric and perchloric acids and the like, as well as organic acids such as tartaric, citric, acetic, succinic, maleic, fumaric and oxalic acids. Preferred pharmaceutically acceptable base addition salts include salts of alkali metals, e.g. sodium or potassium; alkaline earth metals, e.g. calcium or magnesium; or complex salts such as ammonium or substituted ammonium salts such as mono-, di- or trialkylammonium salts or mono-, di- or trihydroxyalkylammonium salts.

The compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 0.5% of the compounds of the invention, the active ingredient, but may be varied depending upon the particular form and may conveniently be between 4% to about 75% of the weight of the unit. The amount of compound present in such composition is such that a suitable dosage will be obtained. Preferred compositions and preparations according to the present invention are prepared so that an oral dosage unit form contains between 1.0-300 mgs of the compounds of the invention.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel®, corn starch and the like; a lubricant such as magnesium strearate or Sterotex®; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavors. Mateials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of the aforesaid compound, but may be varied between 0.5 and about 30% of the weight thereof. The amount of active compound in such compositions is such that a suitable dosage will be obtained. Preferred compositions and preparations according to the present invention are prepared so that a parenteral dosage unit contains between 0.5 to 100 mgs of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

For the purpose of topical administration, the active compounds of the invention may be incorporated into a solution, suspension, ointment, cream, gel, aerosol or salve. These preparations should contain at least 0.1% of active compound but may be varied to be between 0.05 and about 20% of the weight thereof. The amount of active compound in such compositions is such that a suitable dosage will be obtained. Preferred topically administered preparations should contain between 0.1 and 10% of active compound.

The topical compositions may also include the following components: water, fixed oils, polyethylene glycols, glycerol, petroleum stearic acid, beeswax, other synthetic solvents or mixtures thereof; antibacterial agents such as benzyl alcohol or methyl paraben; antioxidants such as α-tocopherol acetate; chelating agents such as ethylenediaminetetraacetic acid (EDTA); buffers such as acetates, citrates or phosphates; emulsifying agent such as polyoxyethylene monooleate and coloring materials and adjuvants such as ferric oxide or talc. The topical preparation can be enclosed in tubes, bottles or jars made of metal, glass or plastic.

Examples of the compounds of this invention or intermediates therefor include:
2-(6,11-Dihydro-11-oxodibenz[b,e]oxepin-2-yl)-N-hydroxy-N-methyl-ethylamine;
2-(6,11-Dihydro-11-oxodibenz[b,e]oxepin-2-yl)-N-hydroxy-N-methylacetamide;
2-(6,11-Dihydro-11-oxodibenz[b,e]oxepin-2-yl)-N-hydroxy-N-(1-methylethyl)acetamide;
2-(6,11-Dihydro-11-oxodibenz[b,e]oxepin-2-yl)-N-ethyl-N-hydroxyacetamide;
N-Cyclohexyl-2-(6,11-dihydro-11-oxodibenz[b,e]oxepin-2-yl)-N-hydroxyacetamide;
2-(6,11-Dihydro-11-oxodibenz[b,e]oxepin-2-yl)-N-ethyl-N-hydroxyethylamine;
2-(6,11-Dihydro-11-oxodibenz[b,e]oxepin-2-yl)-N-hydroxy-N-(2-methylethyl) ethylamine;
2-(6,11-Dihydro-11-oxodibenz[b,e]oxepin-2-yl)-N-hydroxy-N-methylpropanamide;
4-[[2-(methoxycarbonyl)phenyl]methoxy]benzenepropionic acid ethyl ester;
2-(6,11-Dihydro-11-oxodibenz[b,e,]oxepin-2-yl)-N-ethyl-N-hydroxypropanamide;
3-(6,11-Dihydro-11-oxodibenz[b,e]oxepin-2-yl)-N-hydroxy-N-methylpropanamide;
3-(6,11-Dihydro-11-oxodibenz[b,e]oxepin-2-yl)propanyl chloride;
3-(6,11-Dihydro-11-oxodibenz[b,e]oxepin-2-yl)-N-hydroxy-N-(1-methylethyl) propanamide;
3-(6,11-Dihydro-11-oxodibenz[b,e]oxepin-2-yl)-N-ethyl-N-hydroxypropanamide;
3-(6,11-Dihydro-11-oxodibenz[b,e]oxepin-2-yl)-N-cyclohexyl-N-hydroxypropanamide;
2-[[4-(3-Hydroxypropyl)phenoxy]methyl]benzoic acid;
2-[[4-(3-Hydroxypropyl)phenoxy]methyl]benzoic acid methyl ester;
3-(6,11-Dihydro-11-oxodibenz[b,e]oxepin-2-yl)propyl trifluoroacetate.
3-(6,11-Dihydro-11-oxodibenz[b,e]oxepin-2-yl)propanol;
3-(6,11-Dihydro-11-oxodibenz[b,e]oxepin-2-yl)-N-hydroxy-N-methyl-1-propylamine;
4-(4,10-Dihydro-10-oxothieno[3,2-c][1]benzoxepin-8-yl)butanol methanesulfonate;
4-(4,10-Dihydro-10-oxothieno[3,2-c][1]benzoxepin-8-yl)-N-hydroxy-N-methyl-1-butylamine;
N-[2-(6,11-Dihydro-11-oxodibenz[b,e]oxepin-2-yl)ethyl]-N-hydroxyacetylacetamide;
2-(6,11-Dihydro-11-oxodibenz[b,e]oxepin-2-yl)acetaldoxime; and
N-[2-(6,11-Dihydro-11-oxidibenz[b,e]oxepin-2-yl)methyl]-N-hydroxyacetamide.

The following examples are for illustrative purposes and are not to be construed as limiting the invention disclosed herein. All temperatures are given in degrees centigrade.

### EXAMPLE 1

### 2-(6,11-Dihydro-11-oxodibenz[b,e]oxepin-2-yl)-N-hydroxy-N-methyl-ethylamine

To a flask was added 25.08 g of potassium *tert*-butoxide and 18.68 g of N-methylhydroxylamine hydrochloride in 700 ml of 95% ethanol with stirring. To the mixture was added 15.02 g of finely ground 2-(6,11-dihydro-11-oxodibenz[b,e]oxepin-2-yl)ethanol methanesulfonate and the stirred suspension was allowed to reflux for 24 hours. The suspension was concentrated to dryness and the solid residue was partitioned against 500 ml of 5% sodium bicarbonate and a total of 600 ml of methylene chloride. The dried (MgSO₄) organic phase was filtered and concentrated to an oil. Evaluation of the crude product (13.44 g) by thin layer analysis (silica gel, ethyl acetate) indicated a mixture, which was separated using preparative High Performance Liquid Chromatography (HPLC hereafter) (Waters Associates Prep LC/System 500, silica gel, ethyl acetate) to give 7.24 g (57%) of pure material. Recrystallization from methanol afforded 2-(6,11-dihydro-11-oxodibenz[b,e]oxepin-2-yl)-N-hydroxy-N-methyl-ethylamine, mp 101-102 °C.

| Analysis: | | | |
|---|---|---|---|
| Calculated for C₁₇H₁₇N O₃: | 72.07%C | 6.05%H | 4.94%N |
| Found: | 71.92%C | 5.87%H | 4.89%N |

### EXAMPLE 2

### 2-(6,11-Dihydro-11-oxodibenz[b,e]oxepin-2-yl)-N-hydroxy-N-methylacetamide

To a flask was added 11.6 g of N-methylhydroxylamine hydrochloride in 250 ml of pyridine. The mixture was stirred to afford a solution, and the flask was chilled with an ice bath. To the stirred solution was added, dropwise over several minutes, a solution of 10.0 g of 2-(6,11-dihydro-11-oxodibenz[b,e]oxepin-2-yl)acetyl chloride in 400 ml of tetrahydrofuran. After the addition was complete, the flask was allowed to equilibrate to room temperature with continued stirring for 24 hours. The suspension was condensed by half using rotary evaporation and was transferred to a separatory funnel. The mixture was partitioned against 300 ml of methylene chloride and 100 ml of 10% HCl (aqueous phase below pH 1). The dried (MgSO₄) organic phase was filtered and concentrated to an oil using rotary evaporation. Thin layer analysis indicated a mixture, which was separated via preparative HPLC to give 4.76 g (45.8%) of pure material. Recrystallization from acetone afforded 2-(6,11-dihydro-11-oxodibenz[b,e]oxepin-2-yl)-N-hydroxy-N-methylacetamide, mp 118-120°C.

| Analysis: | | | |
|---|---|---|---|
| Calculated for C₁₇H₁₅NO₄: | 68.68%C | 5.09%H | 4.71%N |
| Found: | 68.77%C | 5.06%H | 4.68%N |

### EXAMPLE 3

### 2-(6,11-Dihydro-11-oxodibenz[b,e]oxepin-2-yl)-N-hydroxy-N-(1-methylethyl)acetamide

To a flask was added 10.0 g of N-isopropylhydroxylamine hydrochloride in 400 ml of dry pyridine. The mixture was stirred to afford a solution, chilled with an ice bath and treated dropwise over several minutes with a solution of 5.72 g of 2-(6,11-dihydro-11-oxodibenz [b,e]oxepin-2-yl)acetyl chloride in 200 ml of dry tetrahydrofuran. The solution was stirred for 24 hours and was allowed to equilibrate to room temperature. The resulting cloudy suspension was condensed to an oil via rotary evaporation and was transferred to a separatory funnel. The product was partitioned against 500 ml of methylene chloride and a sufficient amount (500 ml) of 10% HCl to acidify the aqueous phase below pH 1. The dried (Na₂SO₄) organic phase was filtered and concentrated to an oil using rotary evaporation. Thin layer analysis indicated a mixture, which was separated via HPLC to give 3.92 g (40.2%) of pure material. Recrystallization from acetone afforded 2-(6,11-dihydro-11-oxodibenz[b,e]oxepin-2-yl)-N-hydroxy-N-(1-methylethyl)acetamide, mp 144-145°C.

| Analysis: | | | |
|---|---|---|---|
| Calculated for C₁₉H₁₉NO₄: | 70.14%C | 5.89%H | 4.30%N |
| Found: | 70.14%C | 5.88%H | 4.24%N |

### EXAMPLE 4

### 2-(6,11-Dihydro-11-oxodibenz[b,e]oxepin-2-yl)-N-ethyl-N-hydroxyacetamide

To a flask was added 4.0 g of N-ethylhydroxylamine hydrochloride in 300 ml of dry pyridine. The mixture was stirred to afford a solution, chilled with an ice bath, and was treated dropwise over several minutes with a solution of 5.72 g of 2-(6,11-dihydro-11-oxodibenz[b,e]oxepin-2-yl)acetyl chloride in 200 ml of dry tetrahydrofuran. The solution was stirred for 24 hours while allowing the bath to equilibrate to room temperature. The resulting suspension was condensed to an oil via rotary evaporation and was then transferred to a separatory funnel. The product was partitioned against 500 ml of methylene chloride and a sufficient amount (250 ml) of 10% HCL to acidify the aqueous phase below pH 1. The organic phase was then washed with 250 ml of water. The dried (Na₂SO₄) organic phase was filtered and concentrated to an oil using rotary evaporation. Thin layer analysis indicated a mixture, which was separated via HPLC to give 3.03 g (49%) of pure material. The product was combined with another lot of identically prepared material, which was found to be pure by thin layer analysis. The total amount of combined material was 4.53 g. Recrystallization from acetone afforded 2-(6,11-dihydro-11-oxodibenz[b,e]-oxepin-2-yl)-N-ethyl-N-hydroxyacetamide, mp 119-121°C.

| Analysis: | | | |
|---|---|---|---|
| Calculated for C₁₈H₁₇NO₄: | 69.44%C | 5.50%H | 4.50%H |
| Found: | 69.63%C | 5.45%H | 4.29%N |

### EXAMPLE 5

### N-Cyclohexyl-2-(6,11-dihydro-11-oxodibenz[b,e]oxepin-2-yl)-N-hydroxyacetamide

To a flask was added 4.0 g of N-cyclohexylhydroxylamine in 400 ml of dry pyridine. The mixture was stirred to afford a solution, chilled with an ice bath, and treated dropwise over several minutes with a solution of 4.86 g of 2-(6,11-dihydro-11-oxodibenz[b,e,]oxepin-2-yl)acetyl chloride in 200 ml of dry tetrahydrofuran. The solution was stirred for 24 hours, while allowing the bath to equilibrate to room temperature. The resulting solution was concentrated to an oil, via rotary evaporation, and was partitioned against 500 ml of methylene chloride and a sufficient amount (250 ml) of 10% HCL to acidify the aqueous phase below pH 1. The organic phase was then washed with 250 ml of distilled water. The dried (Na₂SO₄) organic phase was filtered and concentrated to an oil using rotary evaporation. Thin layer analysis indicated a mixture, which was separated via preparative HPLC to give 3.14 g (51%) of the product. Recrystallization from acetone afforded N-cyclohexyl-2-(6,11-dihydro-11-oxodibenz[b,e]oxepin-2-yl)-N-hydroxyacetamide, mp 130-131°C.

| Analysis: | | | |
|---|---|---|---|
| Calculated for C₂₂H₂₃NO₄: | 72.31%C | 6.34%H | 3.83%N |
| Found: | 72.12%C | 6.15%H | 3.58%N |

### EXAMPLE 6

### 2-(6,11-Dihydro-11-oxodibenz[b,e]oxepin-2-yl)-N-ethyl-H-hydroxyethylamine

To a flask was added 4.48 g of potassium *tert*-butoxide and 3.95 g of N-ethylhydroxylamine hydrochloride in 500 ml of absolute ethanol. The suspension was stirred, and precipitation of potassium chloride was noted. To the suspension was added 3.32g of finely ground 2-(6,11-dihydro-11-oxodibenz[b,e]oxepin-2-yl)ethanol methanesulfonate, and the slurry was allowed to reflux for 4 1/2 hours. To the product mixture was added 500 ml of water and the solution was concentrated via rotary evaporation to remove ethanol. The resulting suspension was transferred to a separatory funnel and partitioned against a total of 600 ml of methylene chloride and 1000 ml of water. The dried (Na₂SO₄) organic phase was filtered and concentrated to an oil using rotary evaporation. Thin layer analysis indicated a mixture, which was separated via preparative HPLC to give 1.24 g (42%) of 2-(6,11-dihydro-11-oxodibenz[b,e]oxepin-2-yl)-N-ethyl-N-hydroxyethylamine, mp 86-88°C.

| Analysis: | | | |
|---|---|---|---|
| Calculated for C₁₈H₁₉NO₃: | 72.71%C | 6.44%H | 4.71%N |
| Found: | 72.45%C | 6.40%H | 4.57%N |

### EXAMPLE 7

### 2-(6,11-Dihydro-11-oxodibenz[b,e]oxepin-2-yl)-N-hydroxy-N-(2-methylethyl)ethylamine

To a flask was added 14.37 g of potassium *tert*-butoxide and 13.08 g of N-isopropylhydroxylamine hydrochloride in 1500 ml of absolute ethanol. The suspension was stirred, and precipitation of potassium chloride was noted. To the suspension was added 10.62 g of finely ground 2-(6,11-dihydro-11-oxodibenz[b,e]oxepin-2-yl)ethanol methanesulfonate, and the slurry was allowed to reflux for 24 hours. To the product mixture was added 500 ml of water and the solution was concentrated via rotary evaporation to remove ethanol. The resulting suspension was transferred to a separatory funnel and partitioned against a total of 500 ml of methylene chloride and 1000 ml of water. The dried (Na₂SO₄) organic phase was filtered and concentrated to an oil using rotary evaporation. Thin layer analysis indicated a mixture, which was separated via preparative HPLC to give 3.20 g (32%) of pure material. Recrystallization from acetonitrile afforded 2-(6,11-dihydro-11-oxodibenz[b,e]oxepin-2-yl)-N-hydroxy-N-(2-methylethyl)ethylamine, mp 121-124°C.

| Analysis: | | | |
|---|---|---|---|
| Calculated for C₁₉H₂₁NO₃: | 73.29%C | 6.80%H | 4.50%N |
| Found: | 73.55%C | 6.52%H | 4.67%N |

### EXAMPLE 8

### 2-(6,11-Dihydro-11-oxodibenz[b,e]oxepin-2-yl)-N-hydroxy-N-methylpropanamide

To a flask was added a solution of a few drops of dimethylformamide and 50.0 g of 2-(6,11-dihydro-11-oxodibenz[b,e,]oxepin-2-yl)propionic acid in methylene chloride, and the solution was chilled. To the cold solution was added dropwise over several minutes, 13.14 ml of thionyl chloride. The solution was intermittently warmed on a steam bath until all gas evolution ceased, and was allowed to stir for 24 hours. The solution was concentrated to an oil *in vacuo* to remove solvents and any unreacted thionyl chloride to give 45.4 g (84%) of the pure acid chloride.

A stirred solution of 16.6g of N-methylhydroxylamine hydrochloride in 400 ml of dry pyridine was chilled and treated dropwise over several minutes with a solution of 15.7 g of 2-(6,11-dihydro-11-oxodibenz[b,e]oxepin-2-yl)propionyl chloride in 500 ml of tetrahydrofuran. The solution was allowed to equilibrate to room temperature with continued stirring. Thin layer analysis (silica gel, 30% hexane in ethyl acetate) indicated reaction completion after 5 hours. Pyridine and tetrahydrofuran were removed *in vacuo* and the resulting mixture was partitioned against a total of 700 ml of methylene chloride and 2000 ml of 10% HCl (pH of aqueous layers <1). The organic phase was washed with 500 ml of water. The dried (Na₂SO₄) organic phase was concentrated *in vacuo* to an oil. Thin layer analysis indicated a mixture, which was separated via preparative HPLC to give 10.58 g (68%) of the product, 2-(6,11-dihydro-11-oxodibenz[b,e]oxepin-2-yl)-N-hydroxy-N-methylpropanamide, as a viscous oil.

| Analysis: | | | |
|---|---|---|---|
| Calculated for C₁₈H₁₇NO₄: | 69.44%C | 5.50%H | 4.50%N |
| Found: | 69.12%C | 5.72%H | 4.06%N |

### EXAMPLE 9

### 4-[[2-(methoxycarbonyl)phenyl]methoxy]benzenepropionic acid ethyl ester

To a dry flask under inert atmosphere was added 97.0 g of 3-(4-hydroxyphenyl)propionic acid, 207 g of potassium carbonate, and 1.0 g (cat.) of sodium iodide in 600 ml of 2-butanone. The suspension was rapidly purged using several vacuum/N₂ cycles. To the stirring suspension was added dropwise over 30 minutes, a solution of 114.5 g of 2-(bromomethyl)benzoic acid methyl ester in 1000 ml of 2-butanone. The resulting white suspension was allowed to reflux for 24 hours, cooled and allowed to stir at room temperature for 1 hour. The solids were removed by vacuum filtration and washed on the funnel. The solution was concentrated to an oil *in vacuo*. The oil was partitioned against a total of 500 ml of methylene chloride and 500 ml of 10% NaOH (pH of aqueous layers > 11), and washed with 800 mL of water. The dried (Na₂SO₄) organic layer was concentrated to an oil *in vacuo*. Thin layer analysis indicated a mixture, which was separated via preparative HPLC to give 109.95 g (64%) of pure 4-[[2-(methoxycarbonyl)phenyl]methoxy]benzenepropionic acid ethyl ester as an oil.

| Analysis: | | |
|---|---|---|
| Calculated for C₂₀H₂₂O₅: | 70.16%C | 6.47%H |
| Found: | 70.17%C | 6.53%H |

### EXAMPLE 10

### 2-(6,11-Dihydro-11-oxodibenz[b,e,]oxepin-2-yl]-N-ethyl-N-hydroxypropanamide

To a flask was added a solution of a few drops of dimethylformamide and 50.0 g of 2-(6,11-dihydro-11-oxodibenz[b,e,]oxepin-2-yl)propionic acid in 500 ml of methylene chloride, and the solution was chilled. To the chilled solution was added dropwise over several minutes, 13.14 ml of thionyl chloride. The solution was intermittently warmed on a steam bath until all gas evolution had ceased, and was than allowed to stir for 24 hours. The solution was concentrated to an oil *in vacuo* to remove solvents and any unreacted thionyl chloride to give 45.4 g (84%) of the pure acid chloride.

A stirring solution of 10.14 g of N-ethylhydroxylamine hydrochloride in 350 ml of dry pyridine was chilled and treated dropwise over several minutes with a solution of 8.0 g of 2-(6,11-dihydro-11-oxodibenz[b,e,]oxepin-2-yl)propionyl chloride in 400 ml of tetrahydrofuran. The solution was allowed to equilibrate to room temperature overnight (16 hours) with continued stirring. The reaction was quenched with 500 ml of water and concentrated *in vacuo* to remove solvents. The product mixture was partitioned against a total of 500 ml of methylene chloride and 1000 ml of 10% HCl (pH of aqueous layers <1). The organic phase was washed with 500 ml of water, dried (Na₂SO₄) and concentrated *in vacuo* to an oil. Thin layer analysis indicated a mixture, which was separated twice via preparative HPLC to give 2.65 g (30%) of 2-(6,11-dihydro-11-oxodibenz[b,e]oxepin-2-yl)-N-ethyl-N-hydroxypropanamide, as an oil.

| Analysis: | | | |
|---|---|---|---|
| Calculated for C₁₉H₁₉NO₄: | 70.14%C | 5.89%H | 4.30%N |
| Found: | 69.64%C | 6.02%H | 4.11%N |

### EXAMPLE 11

### 3-(6,11-Dihydro-11-oxodibenz[b,e]oxepin-2-yl)-N-hydroxy-N-methylpropanamide

A stirring solution of 12.58 g of N-methylhydroxylamine hydrochloride in 300 ml of dry pyridine was chilled, degassed using several vacuum/N₂ cycles, and treated dropwise over several minutes with a solution of 9.06 g of 3-(6,11-dihydro-11-oxodibenz[b,e]oxepin-2-yl)propanyl chloride in 200 ml of dry tetrahydrofuran. The solution was allowed to equilibrate to room temperature overnight. The reaction was quenched with 200 ml of water, concentrated *in vacuo* to remove solvents, and partitioned against a total of 200 ml of methylene chloride and 200 ml of 10% hydrochloric acid (pH of the resulting aqueous phase was below 1). The organic phase was washed with 500 mL of water, dried (Na₂SO₄), and concentrated to an oil *in vacuo*. Thin layer analysis indicated mixture a which was separated via HPLC eluting with 50% methylene chloride in tetrahydrofuran to give 5.3 g of material. This material was again separated via HPLC under similar conditions using acetonitrile as eluant, to give 3.62 g (39%) of 3-(6,11-dihydro-11-oxodibenz[b,e]oxepin-2-yl)-N-hydroxy-N-methylpropanamide, as an oil.

| Analysis: | | | |
|---|---|---|---|
| Calculated for C₁₈H₁₇NO₄: | 69.44%C | 5.50%H | 4.50%N |
| Found: | 69.24%C | 5.62%H | 4.50%N |

### EXAMPLE 12

### 3-(6,11-Dihydro-11-oxodibenz[b,e]oxepin-2-yl)propanyl chloride

To a flask was added a few drops of N,N-dimethylformamide and 35.25 g of 3-(6,11-dihydro-11-oxodibenz[b,e]oxepin-2-yl)propanoic acid in 500 ml of dry methylene chloride. The chilled stirring suspension was treated dropwise over several minutes with 11.0 ml of thionyl chloride. The suspension was warmed intermittently on a steam bath until all gas evolution ceased. The resulting solution was then allowed to reflux for 15 minutes and was allowed to stand at room temperature under nitrogen atmosphere overnight. The solution was concentrated *in vacuo* to an oil. The oil was dissolved in 500 ml of methylene chloride and was treated with another 11.0 ml of thionyl chloride. The solution was allowed to reflux for 3 hours and was again concentrated to an oil *in vacuo*. The oil still contained a significant amount of crystalline impurity, which was filtered away after triturating with a hexane/ether solution. The supernatant was concentrated to an oil *in vacuo* which solidified overnight in refrigeration. The purified solids were recrystallized from three portions of cyclohexane to give 25.03 g (67%) of 3-(6,11-dihydro-11-oxodibenz[b,e]oxepin-2-yl)propanyl chloride, mp 69-72°C.

| Analysis: | | |
|---|---|---|
| Calculated for C₁₇H₁₃ClO₃: | 67.89%C | 4.36%H |
| Found: | 68.05%C | 4.61%H |

### EXAMPLE 13

### 3-(6,11-Dihydro-11-oxodibenz[b,e]oxepin-2-yl)-N-hydroxy-N-(1-methylethyl)propanamide

A stirring solution of 11.58 g of N-isopropylhydroxylamine hydrochloride in 300 ml of dry pyridine was chilled, degassed using several vacuum/N₂cycles, and treated dropwise over several minutes with a solution of 7.54 g of 3-(6,11-dihydro-11-oxodibenz[b,e]oxepin-2-yl)propanyl chloride in 200 ml of dry tetrahydrofuran. The solution was allowed to equilibrate to room temperature overnight. The reaction was quenched with 200 ml of water, concentrated *in vacuo* to remove solvents, and partitioned against a total of 300 ml of methylene chloride and 400 ml of 10% hydrochloric acid (pH of the resulting aqueous phase was below 1). The organic phase was washed with 500 ml of water, dried (Na₂SO₄), and concentrated to an oil *in vacuo*. Thin layer analysis indicated a mixture that was separated via HPLC to give 3.78 g of an oil, which crystallized upon standing at room temperature. The purified solids were recrystallized from acetone/cyclohexane to give 2.50 g (29.4%) of 3-(6,11-dihydro-1-oxo-dibenz[b,e]oxepin-2-yl)-N-hydroxy-N-(1-methylethyl)propanamide, mp 117.5-118°C.

| Analysis: | | | |
|---|---|---|---|
| Calculated for C₂₀H₂₁NO₄: | 70.78%C | 6.24%H | 4.13%N |
| Found: | 70.97%C | 6.30%H | 4.14%N |

### EXAMPLE 14

### 3-(6,11-Dihydro-11-oxodibenz[b,e]oxepin-2-yl)-N-ethyl-N-hydro xypropanamide

A stirring solution of 8.98 g of N-ethylhydroxylamine hydrochloride in 300 ml of dry pyridine was chilled, degassed using several vacuum/N₂ cycles, and treated dropwise over several minutes with a solution of 9.00 g of 3-(6,11-dihydro-11-oxodibenz[b,e]oxepin-2-yl)propanyl chloride in 200 ml of dry tetrahydofuran. The solution was allowed to equilibrate to room temperature overnight. The reaction was quenched with 200 ml of water, concentrated *in vacuo* to remove solvents, and partitioned against a total of 250 ml of methylene chloride and 600 ml of 10% hydrochloric acid (pH of the resulting aqueous phase was below 1). The organic phase was washed with 500 ml of water, dried (Na₂SO₄), and concentrated to an oil *in vacuo*. The oil was triturated with 25% hexane/ethyl acetate to give 6.11 g of solids, which were recrystallized from hexane/ethyl acetate to give 4.56 g (46.8%) of 3-(6,11-dihydro-11-oxodibenz[b,e]oxepin-2-yl)-N-ethyl-N-hydroxypropanamide, mp 120°C.

| Analysis: | | | |
|---|---|---|---|
| Calculated for C₁₉H₁₉NO₄: | 70.14%C | 5.89%H | 4.30%N |
| Found: | 69.97%C | 5.85%H | 4.27%N |

### EXAMPLE 15

### 3-(6,11-Dihydro-11-oxodibenz[b,e]oxepin-2-yl)-N-cyclohexyl-N-hydroxypropanamide

A stirring solution of 20.5 g of N-cyclohexylhydroxylamine hydrochloride in 300 ml of dry pyridine was chilled, degassed using several vacuum/N₂ cycles and was treated dropwise over several minutes with a degassed solution of 13.50 g of 3-(6,11-dihydro-11-oxodibenz[b,e]oxepin-2-yl)propanyl chloride in 300 ml of dry tetrahydrofuran. The resulting solution was allowed to equilibrate to room temperature overnight. The reaction was quenched with 200 ml of water, stirred for an additional 15 minutes, and was concentrated *in vacuo* to remove solvents. The resulting suspension was partitioned against a total of 300 ml of methylene chloride and 300 ml of 10% hydrochloric acid (pH of the resulting aqueous phase was below 1). The organic phase was washed with 500 ml of water, dried (Na₂SO₄), and concentrated to an oil *in vacuo*. The oil was triturated to an amorphous solid with 25% hexane/ethyl acetate to give 5.26 g (31%) of 3-(6,11-dihydro-11-oxodibenz[b,e]oxepin-2-yl)-N-cyclohexyl-N-hydroxypropanamide, m.p. 146-147°C.

| Analysis: | | | |
|---|---|---|---|
| Calculated for C₂₃H₂₅NO₄: | 72,80%C | 6.64%H | 3.69%N |
| Found: | 72.77%C | 6.65%H | 3.69%N |

### EXAMPLE 16

### 2-[[4-(3-Hydroxypropyl)phenoxy]methyl]benzoic acid

A stirring solution of 127.1 g of potassium hydroxide in 500 ml of 95% ethanol was treated portionwise over several minutes with 59.4 g of 2-[[4-(3-hydroxypropyl)phenoxy]methyl]benzoic acid methyl ester. The resulting solution was allowed to reflux overnight with continued stirring. The product solution was poured into 900 ml of water and the solution was acidified to below pH 2 with approximately 200 ml of concentrated hydrochloric acid. The resulting suspension was transferred to a separatory funnel and was partitioned against a total of 1000 ml of ethyl acetate. The primary aqueous phase was discarded and the organic phase was washed with several 500 ml aliquots of water until the pH of the final aqueous phase was neutral. The dried (Na₂SO₄) organic phase was concentrated *in vacuo* to a solid which was recrystallized from ethanol/water to give 49.13 g (87%) of 2-[[4-(3-hydroxypropyl)phenoxy]methyl]benzoic acid, mp 139-140°C.

| Analysis: | | |
|---|---|---|
| Calculated for C₁₇H₁₈O₄: | 71.31%C | 6.34%H |
| Found: | 71.42%C | 6.27%H |

### EXAMPLE 17

### 2-[[4-(3-Hydroxypropyl)phenoxy]methyl]benzoic acid methyl ester

A stirring degassed suspension of 57.9 g of 3-(4-hydroxyphenyl)propanol, 157 g of potassium carbonate, and a catalytic amount of potassium iodide in 500 ml of 2-butanone was treated dropwise over several minutes with a solution of 87.6 g of 2-(bromomethyl)benzoic acid methyl ester in 500 ml of 2-butanone. The resulting mixture was allowed to reflux for 24 hours and the resulting suspension was then stirred at room temperature for an additional 6 hours. The solids were removed by filtration and the product solution was concentrated to an oil *in vacuo*. The oil was partitioned against a total of 500 ml of methylene chloride and 1500 ml of 10% aqueous sodium hydroxide. The resulting pH of the final aqueous phase was above 12. The organic phase was washed with 500 ml of water, dried (Na₂SO₄), and concentrated to an oil *in vacuo*. Thin layer analysis indicated a mixture which was separated via HPLC. Poor initial separation gave a mixture, which was separated using 60% hexane in ethyl acetate as eluent to give 68.71 g (60%) of 2-[[4-(3-hydroxypropyl)phenoxy]methyl]benzoic acid methyl ester as an oil, which solidified to an amorphous material upon standing at room temperature, mp 50.5-52°C.

| Analysis: | | |
|---|---|---|
| Calculated for C₁₈H₂₀O₄: | 71.98%C | 6.71%H |
| Found: | 72.04%C | 6.61%H |

### EXAMPLE 18

### 3-(6,11-Dihydro-11-oxodibenz[b,e]oxepin-2-yl)propyl trifluoroacetate

A stirring suspension of 46.71 g of 2-[[4-(3-hydroxypropyl)phenoxy]methyl] benzoic acid in 500 ml of dry methylene chloride was treated dropwise over several minutes with 82.70 g of trifluoroacetic acid anhydride. The resulting solution was allowed to reflux for 4 hours and was then cooled to ambient temperature and treated with 200 ml of water. The biphasic mixture was acidified with 200 ml of 10% aqueous hydrochloric acid, transferred to a separatory funnel, and the aqueous phase was discarded. The organic phase was partitioned against two 200 ml aliquots of 5% aqueous sodium bicarbonate until the pH of the final aqueous phase was above 8. The organic phase was washed with 500 ml of water, dried (Na₂SO₄), and concentrated *in vacuo* to an oil which solidified upon standing a room temperature. A 5.5 g aliquot of this material was recrystallized from 2,2,4-trimethylpentane (*i*-octane) to give 3-(6,11-dihydro-11-oxo-dibenz[b,e]oxepin-2-yl)propyl trifluoroacetate, mp 40.5 - 41.0°C.

| Analysis: | | |
|---|---|---|
| Calculated for C₁₉H₁₅F₃O₄: | 62.64%C | 4.15%H |
| Found: | 62.84%C | 4.18%H |

### EXAMPLE 19

### 3-(6,11-Dihydro-11-oxodibenz[b,e]oxepin-2-yl)propanol

A stirring degassed solution of 54.03 g of 3-(6,11-dihydro-11-oxodibenz[b,e]oxepin-2-yl)propyl trifluoroacetate in 300 ml of acetone was treated over several minutes with 250 ml of 10% aqueous hydrochloric acid. The resulting solution was allowed to reflux for 24 hours, cooled to room temperature, and was then concentrated *in vacuo* to remove acetone. The biphasic mixture was partitioned against a total of 1000 ml of methylene chloride and 1500 ml of 5% aqueous sodium bicarbonate until the pH of the final aqueous phase was above 8. The organic phase was washed with 500 ml of water, dried (Na₂SO₄), and concentrated *in vacuo* to an oil. Thin layer analysis indicated a mixture, which was separated via HPLC (silica gel, eluted with 50% ethyl acetate in hexane) to give 35.77 g (90.2%) of pure 3-(6,11-dihydro-11-oxodibenz[b,e]oxepin-2-yl)propanol, as an oil.

| Analysis: | | |
|---|---|---|
| Calculated for C₁₇H₁₆O₃: | 76.10%C | 6.01%H |
| Found: | 76.17%C | 6.13%H |

### EXAMPLE 20

### 3-(6,11-Dihydro-11-oxodibenz[b,e]oxepin-2-yl)-N-hydroxy-N-methyl-1-propylamine

A degassed stirring solution of 32.0 g of 3-(6,11-dihydro-11-oxodibenz[b,e]oxepin-2-yl)propanol in 500 ml of sieve dried pyridine was treated dropwise at 0°C over several minutes with 41.27 of methanesulfonyl chloride. The resulting solution was allowed to equilibrate to room temperature overnight. The resulting solution was partitioned against a total of 1300 ml of methylene chloride and 2750 ml of 10% aqueous hydrochloric acid. The pH of the final aqueous phase was below 1. The organic phase was washed with 950 ml of water and was dried with 950 ml of saturated aqueous sodium chloride solution. The dried (Na₂SO₄) organic phase was filtered over MgSO₄ and concentrated *in vacuo* to a viscous oil. The total yield of pure product was 26.03 g (63%) of 3-(6,11-dihydro-11-oxodibenz[b,e]oxepin-2-yl)propanol methanesulfonate.

To a stirring solution of 13.06 g of N-methylhydroxylamine hydrochloride in 250 ml of absolute ethanol was added 17.48 g of potassium *tert*-butoxide. To the resulting suspension was added 9.0 g of 3-(6,11-dihydro-11-oxodibenz[b,e]oxepin-2-yl)propanol methanesulfonate. The resulting product suspension was allowed to reflux for 24 hours and was then cooled to room temperature. The suspension was filtered to remove potassium chloride, and the mother liquor was then treated with 200 ml of water and concentrated *in vacuo* to remove ethanol. The resulting mixture was partitioned against a total of 450 ml of methylene chloride and 250 ml of water. The organic phase was washed with 250 ml of saturated sodium chloride solution, dried (Na₂SO₄), filtered and concentrated *in vacuo* to a viscous oil which solidified upon triturating the oil, with 10 ml of ethyl acetate and 3 drops of hexane and refrigerating. Thin layer analysis of the solids indicated a mixture, which was separated via HPLC (silica gel). The purified oil solidified upon standing to give 4.03 g (52%) of 3-(6,11-dihydro-11-oxodibenz[b,e]oxepin-2-yl)-N-hydroxy-N-methyl-1-propylamine, m.p. 113-114.5°C.

| Analysis: | | | |
|---|---|---|---|
| Calculated for C₁₈H₁₉NO₃: | 72.71%C | 6.44%H | 4.71%N |
| Found: | 72.67%C | 6.36%H | 4.70%N |

### EXAMPLE 21

### 4-(4,10-Dihydro-10-oxothieno[3,2-c][1]benzoxepin-8-yl)butanol methanesulfonate

A chilled starting solution of 9.5 g of 4-(4,10-dihydro-10-oxothieno[3,2-c][1]benzoxepin-8-yl)butanol in 200 ml of sieve dried pyridine was treated dropwise with 3.80 g of methanesulfonyl chloride and the addition funnel was rinsed with a few ml of methylene chloride. The stirring solution was allowed to equilibrate to room temperature overnight with the bath in place. The resulting solution was poured into 750 ml of water and was allowed to stand for 15 min. The solids were collected by filtration, and dried *in vacuo* overnight at 42°C. Concentration of the mother liquor produced additional solids which were combined with the first crop and dried to give a total of 9.09 g (75%) of crude material. A 2.0 g portion of the product was recrystallized from hexane/ethyl acetate to give 1.3 g (49%) of 4-(4,10-dihydro-10-oxothieno[3,2-c][1]benzoxepin-8-yl)butanol methanesulfonate, m.p. 97-98°C.

| Analysis: | | | |
|---|---|---|---|
| Calculated for C₁₇H₁₈O₅S₂: | 55.72%C | 4.95%H | 17.50%S |
| Found: | 56.28%C | 5.03%H | 17.12%S |

### EXAMPLE 22

### 4-(4,10-Dihydro-10-oxothieno[3,2-c][1]benzoxepin-8-yl)-N-hydroxy-N-methyl-1-butylamine

To a stirring solution of 4.77 g of N-methylhydroxylamine hydrochloride in 100 ml of absolute ethanol was added 6.38 g of potassium *tert*-butoxide. To the resulting suspension was added 6.98 g of 4-(4,10-dihydro-10-oxothieno[3,2-c][1]benzoxepin-8-yl)butanol methanesulfonate, and an additional 150 ml of absolute ethanol. The suspension was allowed to reflux for 24 hours and was then cooled to room temperature. The suspension was filtered to remove potassium chloride and the filter cake was washed with absolute ethanol. The resulting solution was concentrated *in vacuo* to a waxy solid. The solid was dissolved in 300 ml of methylene chloride and partitioned against 200 ml of water. The organic phase was dried with 200 ml of saturated aqueous sodium chloride, collected, dried again (Na₂SO₄), filtered, and concentrated *in vacuo* to an oil. The oil solidified upon standing, and was recrystallized from isopropanol to give 3.09 (51%) of 4-(4,10-dihydro-10-oxothieno[3,2-c][1]benzoxepin-8-yl)-N-hydroxy-N-methyl-1-butylamine, m.p. 107-108°C.

| Analysis: | | | |
|---|---|---|---|
| Calculated for C₁₇H₁₉NO₃S: | 64.33%C | 6.03%H | 4.41%N |
| Found: | 64.31%C | 6.04%H | 4.30%N |

### EXAMPLE 23

### 2-(6,11-dihydro-11-oxodibenz[b,e]oxepin-2-yl)acetaldoxime

A solution of 2-(6,11-dihydro-11-oxodibenz[b,e]oxepin-2-yl)acetyl chloride (15.00 g) in 150 ml of dry diglyme was stirred at -78°C under nitrogen as lithium tri-tert-butoxyaluminohydride (104.6 ml of a 0.5 M solution in diglyme) was added slowly dropwise over one hour. The reaction was stirred ten minutes at -78°C and then quenched with 3 ml of glacial acetic acid added slowly dropwise. After warming the reaction to room temperature 50 ml of pyridine and 2 equiv. of hydroxylamine hydrochloride (7.27 g) was added and the slurry stirred at 50°C for two hours. The solvent was evaporated using high vacuum and the residue taken up in chloroform and 1N HCl. The layers were separated and the aqueous phase extracted with chloroform. The combined organic phases were dried (MgSO₄), filtered, and evaporated. The residue was purified by flash chromatography (silica; 5:2 hex-EtOAc) and recrystallized from ethyl acetate-hexane to give 2-(6,11-dihydro-11-oxodibenz[b,e]oxepin-2-yl)acetaldoxime, as crystals, m.p. 132-134°C.

| Analysis: | | | |
|---|---|---|---|
| Calculated for C₁₆H₁₃NO₃: | 71.90%C | 4.90%H | 5.24%N |
| Found: | 71.75%C | 4.64%H | 5.20%N |

### EXAMPLE 24

### N-[2-(6,11-dihydro-11-oxodibenz[b,e]oxepin-2-yl)methyl]-N-hydroxacetylacetamide

A solution of 2-(6,11-dihydro-11-oxodibenz[b,e]oxepin-2-yl)-N-hydroxymethylamine (5.85 g), triethylamine (6.96 g) and 100 ml of dry dichloromethane was stirred at 0°C under nitrogen as acetyl chloride (3.96 g) was added dropwise. The reaction was stirred at 0°C for one hour and then poured over 100 ml of 2N HCl. The layers were separated and the aqueous phase extracted with dichloromethane. The combined organic phases were dried (Na₂SO₄), filtered, and evaporated. The residue was purified by flash chromatography (silica; 2:1 hex-EtOAc) and recrystallized from ether-hexane to give N-[2-(6,11-dihydro-11-oxodibenz[b,e]oxepin-2-yl)methyl]-N-hydroxacetylacetamide, as crystals, m.p. 107-109°C.

| Analysis: | | | |
|---|---|---|---|
| Calculated for C₁₉H₁₇NO₅: | 67.25%C | 5.05%H | 4.13%N |
| Found: | 67.12%C | 4.88%H | 4.09%N |

### EXAMPLE 25

### N-[2-(6,11-Dihydro-11-oxodibenz[b,e]oxepin-2-yl)methyl]-N-hydroxyacetamide

A solution of N-[2-(6,11-dihydro-11-oxodibenz[b,e]oxepin-2-yl)methyl]-N-hydroxyacetylacetamide (2.40 g) in 120 ml of isopropanol was stirred at ambient temperature while 7.0 equiv. of lithium hydroxide monohydrate (2.08 g) in 50 ml of water was added. The reaction was stirred one hour and the isopropanol was evaporated. The residue was partitioned between 2N HCl and ether. The layers were separated and the aqueous phase extracted with additional ether. The combined organic layers were dried (Na₂SO₄), filtered, and evaporated. The residue was purified by flash chromatography (silica; 2:1 ethyl acetate-hexane) and recrystallized from ethyl acetate-hexane to give 1.1 g (52%) of N-[2-(6,11-dihydro-11-oxodibenz[b,e]oxepin-2-yl)methyl]-N-hydroxyacetamide, m.p. 129-130°C.

| Analysis: | | | |
|---|---|---|---|
| Calculated for C₁₇H₁₅NO₄: | 68.68%C | 5.09%H | 4.71%N |
| Found: | 68.56%C | 5.16%H | 4.64%N |

## Claims (Claims for the following Contracting State(s): AT, BE, CH, DE, DK, FR, GB, IT, LI, LU, NL, SE)

1. A compound of the formula 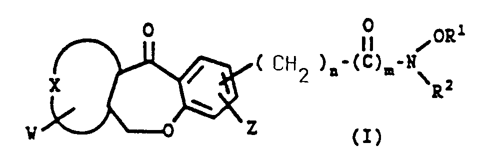 where X together with the carbon atoms to which it is attached forms a benzene or thiophene ring; W and Z are independently hydrogen, halogen, C₁₋₈ alkyl or trifluoromethyl; R¹ is hydrogen, C₁₋₈ alkyl, or acyl; R² is C₁₋₈ alkyl, cycloalkyl, aryl-C₁₋₈ alkyl or acyl; m is 0 or 1 and n is 0 to 12 and the pharmaceutically acceptable salts thereof.

2. A compound as defined in claim 1, where X together with the carbon atoms to which it is attached forms a benzene ring.

3. A compound as defined in claim 2, where R¹ is hydrogen or acyl, R² is C₁₋₈ alkyl, cycloalkyl or acyl, n is 1 to 3 and W and Z are both hydrogen.

4. The compound as defined in claim 1 which is 2 -(6,11-dihydroxy-11-oxodibenz[b,e]oxepin-2-yl)-N-hydroxy-N-methyl-ethylamine.

5. The compound as defined in claim 1 which is 3-(6,11-dihydro-11-oxodibenz[b,e]oxepin-2-yl)-N-hydroxy-N-methyl-propanamide or a pharmaceutically acceptable salt thereof.

6. The compound as defined in claim 1 which is N-cyclohexyl-2-(6,11-dihydro-11-oxo-dibenz[b,e]oxepin-2-yl)-N-hydroxy-acetamide or a pharmaceutically acceptable salt thereof.

7. The compound as defined in claim 1 which is 2-(6,11-dihydro-11-oxodibenz[b,e]oxepin-2-yl)-N-ethyl-N-hydroxy-propanamide or a pharmaceutically acceptable salt thereof.

8. The compound as defined in claim 1 which is 3-(6,11-dihydro-11-oxodibenz[b,e]oxepin-2-yl)-N-cyclohexyl-N-hydroxypropanamide or a pharmaceutically acceptable salt thereof.

9. A compound of the formula Ia 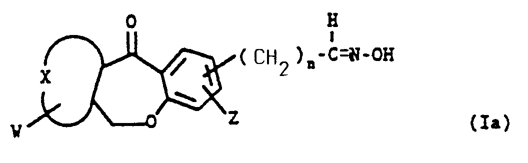 where W, X, Z and n are as previously defined in claim 1.

10. A pharmaceutical composition which comprises as the active ingredient a compound as defined in claim 1 and a pharmaceutically acceptable carrier therefor.

11. Use of a compound as defined in claim 1 for the preparation of a medicament having analgesic or topically antiinflammatoric activity.

12. A process for the preparation of a compound as defined in claim 1, which comprises
a) reacting a compound of formula II 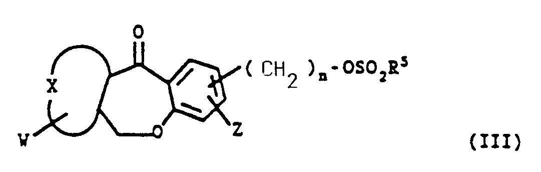 where W, X and Z are as defined in claim 1, n is as defined in claim 1, but is not 0, R⁵ is C₁₋₈ alkyl, with the salt of an N-C₁₋₈ alkylhydroxylamine or N-cycloalkylhydroxylamine to provide a compound of the formula I, where W, X, Z and n are as defined above, m is 0, R¹ is hydrogen and R² is C₁₋₈ alkyl or cycloalkyl, or
b) reacting a compound of formula VII 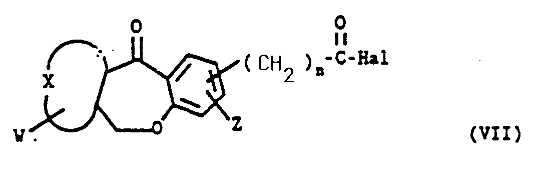 where W, X, Z and n are as defined in claim 1 and Hal is halogen with the salt of an N-C₁₋₈ alkylhydroxylamine or N-cycloalkylhydroxylamine to provide a compound of the formula I, where W, X, Z and n are as defined above, m is 1, R¹ is hydrogen and R² is C₁₋₈ alkyl or cycloalkyl,
c) or reacting a compound of the formula XX 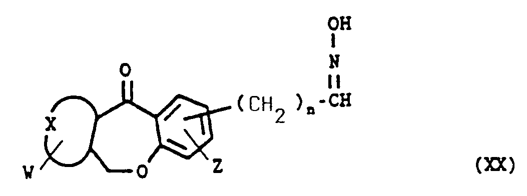 where W, X, Z and n are as defined in claim 1, with a reducing agent to provide a compound of the formula XXI where W, X, Z and n are as defined, m is 0, R¹ is hydrogen and R² is hydrogen, and
d) reacting the compound of the formula XXI as obtained in step c) 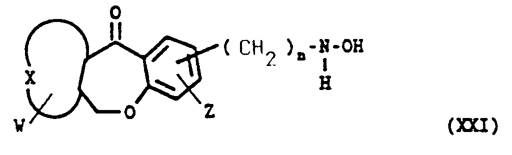 where W, X, Z and n are as defined in claim 1, with an acylating agent to provide a compound of the formula I where W, X, Z and n are as defined, m is 0 and R¹ and R² are both acyl.

## Claims (Claims for the following Contracting State(s): ES, GR)

1. A process for the preparation of a compound of the formula I 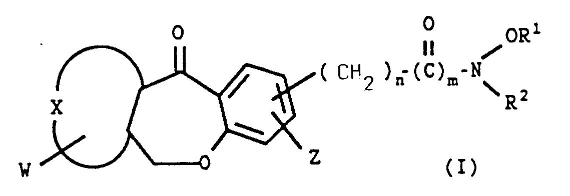 where X together with the carbon atoms to which it is attached forms a benzene or thiophene ring; W and Z are independently hydrogen, halogen, C₁₋₈ alkyl or trifluoromethyl; R¹ is hydrogen, C₁₋₈ alkyl, or acyl; R² is C₁₋₈ alkyl, cycloalkyl, aryl-C₁₋₈ alkyl or acyl; m is 0 or 1 and n is 0 to 12 and the pharmaceutically acceptable salts thereof which comprises
a) reacting a compound of formula II 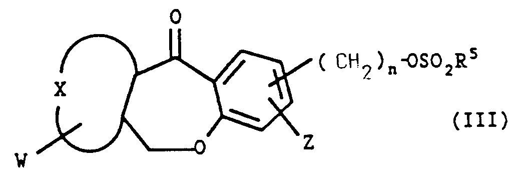 where W, X and Z are as defined in claim 1, n is 1, as defined above, but is not 0, R⁵ is C₁₋₈ alkyl, with an N-C₁₋₈ alkylhydroxylamine hydrochloride or N-cycloalkylhydroxylamine hydrochloride to provide a compound of the formula I, where W, X, Z and n are as defined above, m is 0, R¹ is hydrogen and R² is C₁₋₈ alkyl or cycloalkyl, or
b) reacting a compound of formula VII 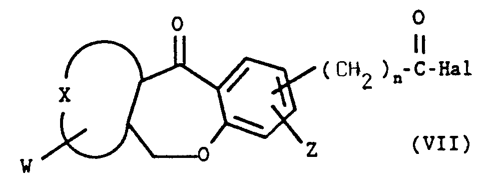 where W, X, Z and n are as defined above and Hal is halogen with the salt of an N-C₁₋₈ alkylhydroxylamine or N-cycloalkylhydroxylamine to provide a compound of the formula I, where W, X, Z and n are as defined above, m is 1, R¹ is hydrogen and R² is C₁₋₈ alkyl or cycloalkyl,
c) or reacting a compound of the formula XX 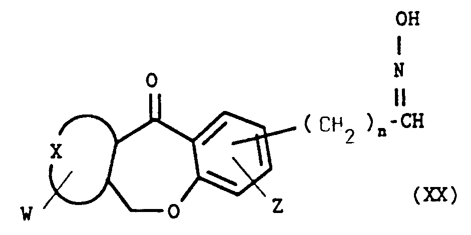 where W, X, Z and n are as defined above, with a reducing agent to provide a compound of the formula XXI where W, X, Z and n are as defined, m is 0, R¹ is hydrogen and R² is hydrogen, and
d) reacting the compound of the formula XXI as obtained in step c) 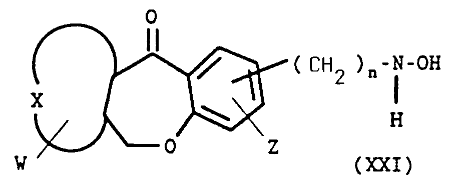 where W, X, Z and n are as defined above, with an acylating agent to provide a compound of the formula I where W, X, Z and n are as defined, m is 0 and R¹ and R² are both acyl.

2. A process as defined in claim 1, where X together with the carbon atoms to which it is attached forms a benzene ring.

3. A process as defined in claim 2, where R¹ is hydrogen or acyl, R² is C₁₋₈ alkyl, cycloalkyl or acyl, n is 1 to 3 and W and Z are both hydrogen.

4. The process as defined in claim 1 wherein 2- (6,11-dihydroxy-11-oxodibenz[b,e]oxepin-2-yl)-N-hydroxy-N-methyl-ethylamine or a pharmaceutically acceptable salt thereof is prepared.

5. The process as defined in claim 1 wherein 3-(6,11-dihydro-11-oxodibenz[b,e]oxepin-2-yl)-N-hydroxy-N-methyl-propanamide or a pharmaceutically acceptable salt thereof is prepared.

6. The process as defined in claim 1 wherein N-cyclohexyl-2-(6,11-dihydro-11-oxo-dibenz[b,e]oxepin-2-yl)-N-hydroxy-acetamide or a pharmaceutically acceptable salt thereof is prepared.

7. The process as defined in claim 1 wherein 2-(6,11-dihydro-11-oxodibenz[b,e]oxepin-2-yl)-N-ethyl-N-hydroxy-propanamide or a pharmaceutically acceptable salt thereof is prepared.

8. The process as defined in claim 1 wherein 3-(6,11-dihydro-11-oxodibenz[b,e]oxepin-2-yl)-N-cyclohexyl-N-hydroxypropanamide or a pharmaceutically acceptable salt thereof is prepared.

9. A compound of the formula Ia 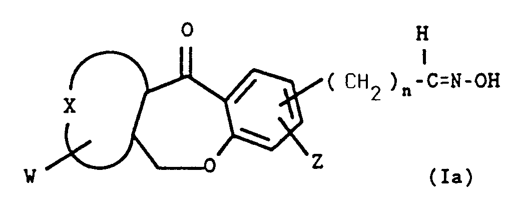 where W, X, Z and n are as previously defined in claim 1.

10. Use of a compound as defined in claim 1 for the preparation of a medicament having analgesic or topically antiinflammatoric activity.

## Patentansprüche (Patentansprüche für folgende(n) Vertragsstaat(en): AT, BE, CH, DE, DK, FR, GB, IT, LI, LU, NL, SE)

1. Verbindung der Formel I 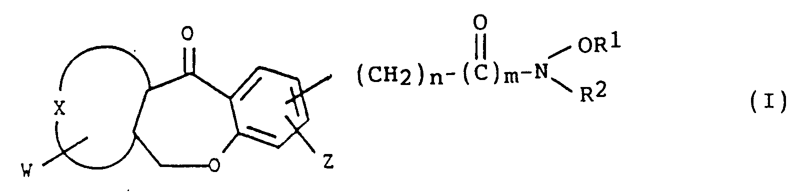 in welcher X zusammen mit den Kohlenstoffatomen, an die es gebunden ist, einen Benzol- oder Thiophenring bildet; W und Z unabhängig voneinander Wasserstoff, Halogen, C₁₋₈-Alkyl oder Trifluormethyl sind, R¹ für Wasserstoff, C₁₋₈-Alkyl oder Acyl steht; R² C₁₋₈-Alkyl, Cycloalkyl, Aryl-C₁₋₈-Alkyl oder Acyl bedeutet; m für 0 oder 1 und n für 0 bis 12 steht; und deren pharmazeutisch verträgliche Salze.

2. Verbindung gemäß Anspruch 1, wobei X zusammen mit den Kohlenstoffatomen, an die es gebunden ist, einen Benzolring bildet.

3. Verbindung gemäß Anspruch 2, wobei R¹ Wasserstoff oder Acyl ist, R² für C₁₋₈-Alkyl, Cycloalkyl oder Acyl steht, n 1 bis 3 bedeutet und W und Z beide Wasserstoff sind.

4. Verbindung gemäß Anspruch 1, bei der es sich um 2-(6,11-Dihydroxy-11-oxodibenz[b,e]oxepin-2-yl)-N-hydroxy-N-methyl-ethylamin handelt.

5. Verbindung gemäß Anspruch 1, bei der es sich um 3-(6,11-Dihydro-11-oxodibenz[b,e]oxepin-2-yl)-N-hydroxy-N-methyl-propanamid oder ein pharmazeutisch verträgliches Salz davon handelt.

6. Verbindung gemäß Anspruch 1, bei der es sich um N-Cyclohexyl-2-(6,11-dihydro-11-oxo-dibenz[b,e]oxepin-2-yl)-N-hydroxy-acetamid oder ein pharmazeutisch verträgliches Salz davon handelt.

7. Verbindung gemäß Anspruch 1, bei der es sich um 2-(6,11-Dihydro-11-oxodibenz[b,e]oxepin-2-yl)-N-ethyl-N-hydroxy-propanamid oder ein pharmazeutisch verträgliches Salz davon handelt.

8. Verbindung gemäß Anspruch 1, bei der es sich um 3-(6,11-Dihydro-11-oxodibenz[b,e]oxepin-2-yl)-N-cyclohexyl-N-hydroxypropanamid oder ein pharmazeutisch verträgliches Salz davon handelt.

9. Verbindung der Formel Ia 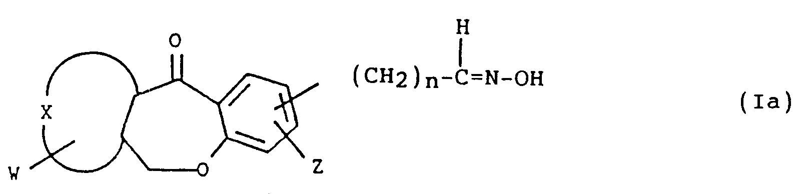 in welcher W, X, Z und n die vorstehend in Anspruch 1 angegebene Bedeutung zukommt.

10. Pharmazeutische Zusammensetzung, die als Wirkstoff eine Verbindung gemäß Anspruch 1 und eine pharmazeutisch verträgliche Trägersubstanz dafür enthält.

11. Verwendung einer Verbindung gemäß Anspruch 1 zur Herstellung eines Medikaments mit analgetischer oder topisch entzündungshemmender Wirksamkeit.

12. Verfahren zur Herstellung einer Verbindung gemäß Anspruch 1, das folgende Schritte umfaßt:
a) Umsetzen einer Verbindung der Formel III 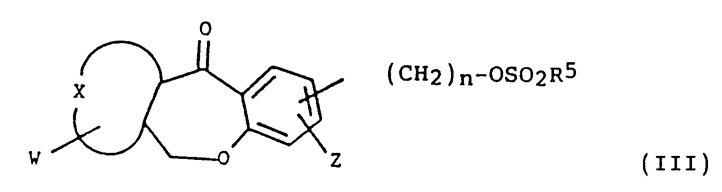 in welcher W, X und Z die in Anspruch 1 angewiesene Bedeutung zukommt, n wie in Anspruch 1 definiert ist, aber nicht 0 ist, und R⁵ für C₁₋₈-Alkyl steht, mit dem Salz eines N-C₁₋₈-Alkylhydroxylamins oder N-Cycloalkylhydroxylamins zur Bildung einer Verbindung der Formel I, in welcher W, X, Z und n wie vorstehend beschrieben definiert sind, m für 0 steht, R¹ Wasserstoff und R² C₁₋₈-Alkyl oder Cycloalkyl bedeutet, oder
b) Umsetzen einer Verbindung der Formel VII 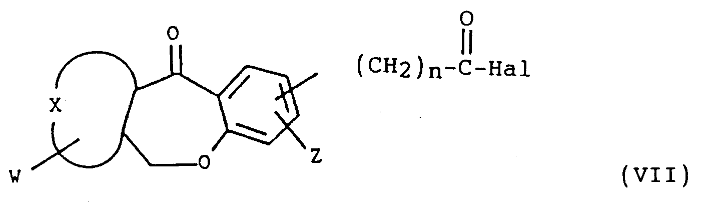 in welcher W, X, Z und n die in Anspruch 1 angewiesene Bedeutung zukommt und Hal Halogen ist, mit dem Salz eines N-C₁₋₈-Alkylhydroxylamins oder N-Cycloalkylhydroxylamins zur Bildung einer Verbindung der Formel I, in welcher W, X, Z und n die weiter oben beschriebene Bedeutung zukommt, m für 1 steht, R¹ Wasserstoff und R² C₁₋₈-Alkyl oder Cycloalkyl bedeutet,
c) oder Umsetzen einer Verbindung der Formel XX, 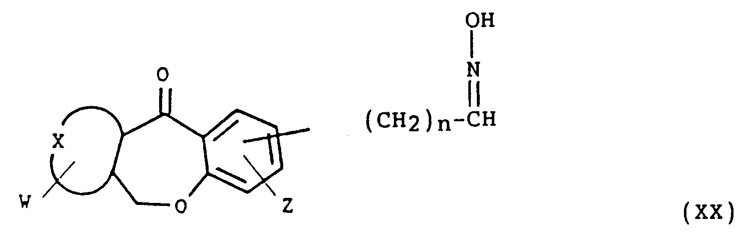 in welcher W, X, Z und n wie in Anspruch 1 beschrieben definiert sind, mit einem Reduktionsmittel zur Bildung einer Verbindung der Formel XXI, in welcher W, X, Z und n vorstehend beschrieben definiert sind, m für 0 steht, R¹ Wasserstoff und R² Wasserstoff bedeutet, und
d) Umsetzen einer Verbindung der Formel XXI, wie sie in Schritt c) erhalten wurde, 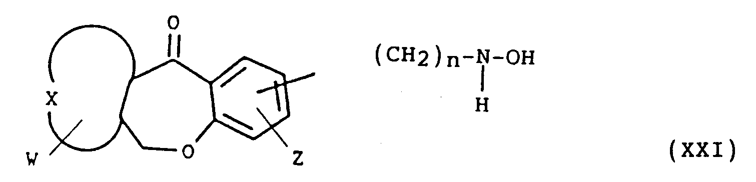 in welcher W, X, Z und n wie in Anspruch 1 beschrieben definiert sind, mit einem Acylierungsmittel zur Bildung einer Verbindung der Formel I, in welcher W, X, Z und n die weiter oben angewiesene Bedeutung zukommt, m für 0 und R¹ und R² beide für Acyl stehen.

## Patentansprüche (Patentansprüche für folgende(n) Vertragsstaat(en): ES, GR)

1. Verfahren zur Herstellung einer Verbindung der Formel I 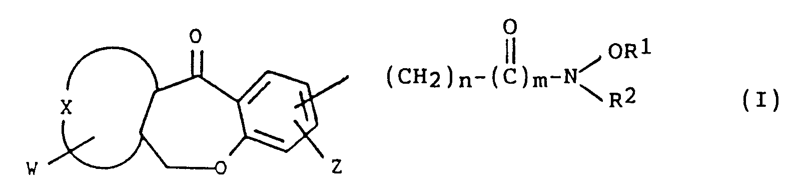 in welcher X zusammen mit den Kohlenstoffatomen, an die es gebunden ist, einen Benzol- oder Thiophenring bildet; W und Z unabhängig voneinander Wasserstoff, Halogen, C₁₋₈-Alkyl oder Trifluormethyl sind, R¹ für Wasserstoff, C₁₋₈-Alkyl oder Acyl steht; R² C₁₋₈-Alkyl, Cycloalkyl, Aryl-C₁₋₈-Alkyl oder Acyl bedeutet; m für 0 oder 1 und n für 0 bis 12 steht; und deren pharmazeutisch verträgliche Salzen;
das folgende Schritte umfaßt:
a) Umsetzen einer Verbindung der Formel III 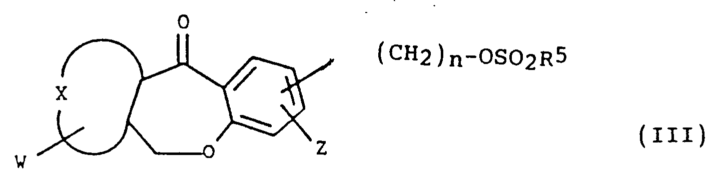 in welcher W, X und Z die vorstehend angewiesene Bedeutung zukommt, n wie vorstehend beschrieben definiert ist, aber nicht 0 ist, und R⁵ für C₁₋₈-Alkyl steht, mit dem Salz eines N-C₁₋₈-Alkylhydroxylaminhydrochlorids oder N-Cycloalkylhydroxylaminhydrochlorids zur Bildung einer Verbindung der Formel I, in welcher W, X, Z und n wie vorstehend beschrieben definiert sind, m für 0 steht, R¹ Wasserstoff und R² C₁₋₈-Alkyl oder Cycloalkyl bedeutet, oder
b) Umsetzen einer Verbindung der Formel VII 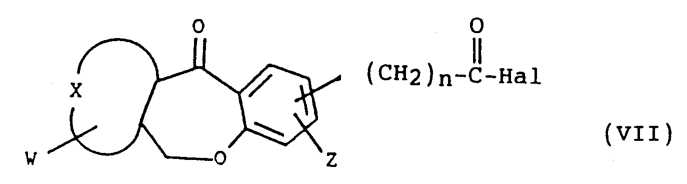 in welcher W, X, Z und n die vorstehend angewiesene Bedeutung zukommt und Hal Halogen ist, mit dem Salz eines N-C₁₋₈-Alkylhydroxylamins oder N-Cycloalkylhydroxylamins zur Bildung einer Verbindung der Formel I, in welcher W, X, Z und n die weiter oben beschriebene Bedeutung zukommt, m für 1 steht, R¹ Wasserstoff und R² C₁₋₈-Alkyl oder Cycloalkyl bedeutet,
c) oder Umsetzen einer Verbindung der Formel XX, 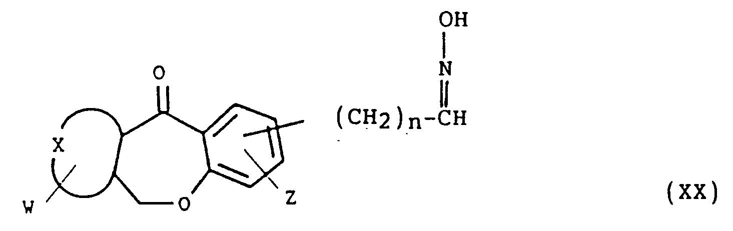 in welcher W, X, Z und n wie vorstehend beschrieben definiert sind, mit einem Reduktionsmittel zur Bildung einer Verbindung der Formel XXI, in welcher W, X, Z und n vorstehend beschrieben definiert sind, m für 0 steht, R¹ Wasserstoff und R² Wasserstoff bedeutet, und
d) Umsetzen einer Verbindung der Formel XXI, wie sie in Schritt c) erhalten wurde, 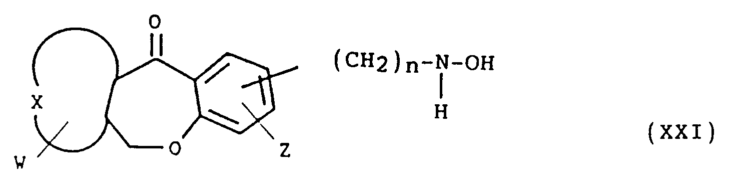 in welcher W, X, Z und n wie vorstehend beschrieben definiert sind, mit einem Acylierungsmittel zur Bildung einer Verbindung der Formel I, in welcher W, X, Z und n die weiter oben angewiesene Bedeutung zukommt, m für 0 und R¹ und R² beide für Acyl stehen.

2. Verfahren gemäß Anspruch 1, wobei X zusammen mit den Kohlenstoffatomen, an die es gebunden ist, einen Benzolring bildet.

3. Verfahren gemäß Anspruch 2, wobei R¹ Wasserstoff oder Acyl ist, R² für C₁₋₈-Alkyl, Cycloalkyl oder Acyl steht, n 1 bis 3 bedeutet und W und Z beide Wasserstoff sind.

4. Verfahren gemäß Anspruch 1, bei dem 2-(6,11-Dihydroxy-11-oxodibenz[b,e]oxepin-2-yl)-N-hydroxy-N-methyl-ethylamin oder ein pharmazeutisch verträgliches Salz davon hergestellt wird.

5. Verfahren gemäß Anspruch 1, bei dem 3-(6,11-Dihydro-11-oxodibenz[b,e]oxepin-2-yl)-N-hydroxy-N-methyl-propanamid oder ein pharmazeutisch verträgliches Salz davon hergestellt wird.

6. Verfahren gemäß Anspruch 1, bei dem N-Cyclohexyl-2-(6,11-dihydro-11-oxo-dibenz[b,e]oxepin-2-yl)-N-hydroxy-acetamid oder ein pharmazeutisch verträgliches Salz davon hergestellt wird.

7. Verfahren gemäß Anspruch 1, bei dem 2-(6,11-Dihydro-11-oxodibenz[b,e]oxepin-2-yl)-N-ethyl-N-hydroxy-propanamid oder ein pharmazeutisch verträgliches Salz davon hergestellt wird.

8. Verfahren gemäß Anspruch 1, bei dem 3-(6,11-Dihydro-11-oxodibenz[b,e]oxepin-2-yl)-N-cyclohexyl-N-hydroxypropanamid oder ein pharmazeutisch verträgliches Salz davon hergestellt wird.

9. Verbindung der Formel Ia 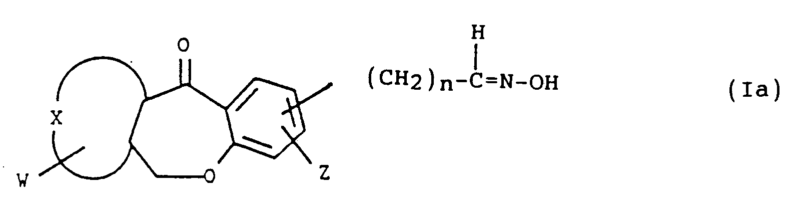 in welcher W, X, Z und n die vorstehend in Anspruch 1 angegebene Bedeutung zukommt.

10. Verwendung einer Verbindung gemäß Anspruch 1 zur Herstellung eines Medikaments mit analgetischer oder topisch entzündungshemmender Wirksamkeit.

## Revendications (Revendications pour l'(les) Etat(s) contractant(s) suivant(s): AT, BE, CH, DE, DK, FR, GB, IT, LI, LU, NL, SE)

1. Composé de formule 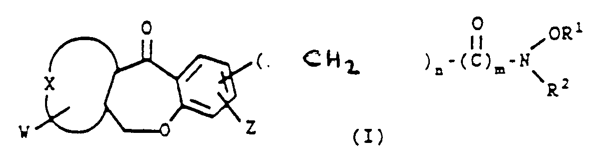 dans laquelle X forme, avec les atomes de carbone auxquels il est fixé, un noyau benzène ou thiophène; W et Z sont, indépendamment, un hydrogène, un halogène, un groupe alkyle en C₁-C₈ ou trifluorométhyle; R¹ est un hydrogène, un groupe alkyle en C₁-C₈ ou acyle; R² est un groupe alkyle en C₁-C₈, cycloalkyle, aryl-alkyle (en C₁-C₈) ou acyle; m a une valeur de 0 ou 1 et n a une valeur de 0 à 12, et ses sels pharmaceutiquement acceptables.

2. Composé tel que défini dans la revendication 1, dans lequel X forme, avec les atomes de carbone auxquels il est fixé, un noyau benzène.

3. Composé tel que défini dans la revendication 2, dans lequel R¹ est un hydrogène ou un groupe acyle, R² est un groupe alkyle en C₁-C₈, cycloalkyle ou acyle, n a une valeur de 1 à 3 et W et Z sont tous deux des atomes d'hydrogène.

4. Composé tel que défini dans la revendication 1, qui est la 2-(6,11-dihydroxy-11-oxodibenzo[b,e]oxépine-2-yl)-N-hydroxy-N-méthyl-éthylamine.

5. Composé tel que défini dans la revendication 1, qui est le 3-(6,11-dihydro-11-oxodibenzo[b,e]oxépine-2-yl)-N-hydroxy-N-méthyl-propanamide, ou un de ses sels pharmaceutiquement acceptables.

6. Composé tel que défini dans la revendication 1, qui est le N-cyclohexyl-2-(6,11-dihydro-11-oxodibenzo[b,e]oxépine-2-yl)-N-hydroxy-acétamide, ou un de ses sels pharmaceutiquement acceptables.

7. Composé tel que défini dans la revendication 1, qui est le 2-(6,11-dihydro-11-oxodibenzo[b,e]oxépine-2-yl)-N-éthyl-N-hydroxy-propanamide, ou un de ses sels pharmaceutiquement acceptables.

8. Composé tel que défini dans la revendication 1, qui est le 3-(6,11-dihydro-11-oxodibenzo[b,e]oxépine-2-yl)-N-cyclohexyl-N-hydroxy-propanamide, ou un de ses sels pharmaceutiquement acceptables.

9. Composé de formule Ia 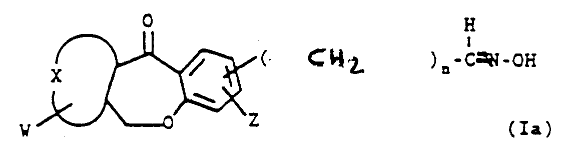 dans laquelle W, X, Z, n et m sont tels que définis précédemment dans la revendication 1, R³ est un hydrogène ou un groupe alkyle en C₁-C₈, R⁴ est un hydrogène, un groupe alkyle en C₁-C₈ ou alcoxy en C₁-C₈, et p a une valeur de 0 ou 1.

10. Composition pharmaceutique qui comprend, comme substance active, un composé tel que défini dans la revendication 1 et un véhicule pharmaceutiquement acceptable pour celui-ci.

11. Utilisation d'un composé tel que défini dans la revendication 1, pour la préparation d'un médicament ayant une activité analgésique ou anti-inflammatoire en topique.

12. Procédé de préparation d'un composé tel que défini dans la revendication 1, qui comprend
a) la réaction d'un composé de formule II 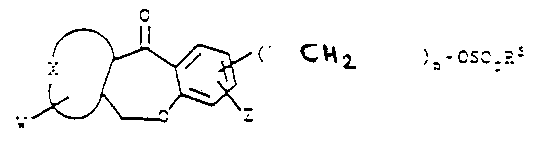 dans laquelle W, X et Z sont tels que définis dans la revendication 1, n est tel que défini dans la revendication 1, mais n'est pas nul, R⁵ est un groupe alkyle en C₁-C₈, avec le sel d'une N-alkyl(en C₁-C₈)hydroxylamine ou d'une N-cycloalkylhydroxylamine, pour donner un composé de formule I, dans laquelle W, X, Z et n sont tels que définis ci-dessus, m est nul, R¹ est un hydrogène et R² est un groupe alkyle en C₁-C₈ ou cycloalkyle, ou
b) la réaction d'un composé de formule VII 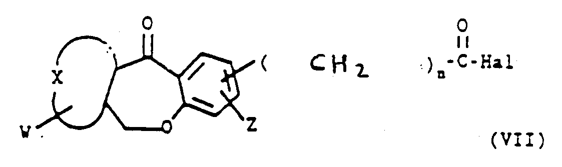 dans laquelle W, X, Z et n sont tels que définis dans la revendication 1 et Hal est un halogène, avec le sel d'une N-alkyl(en C₁-C₈)hydroxylamine ou d'une N-cycloalkylhydroxylamine, pour donner un composé de formule I, dans laquelle W, X, Z et n sont tels que définis ci-dessus, m est égal à 1, R¹ est un hydrogène et R² est un groupe alkyle en C₁-C₈ ou cycloalkyle, ou
c) la réaction d'un composé de formule XX 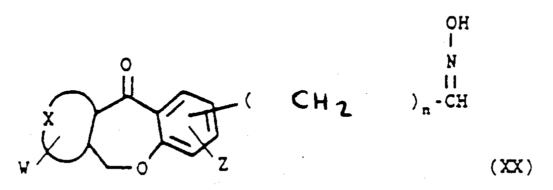 dans laquelle W, X, Z et n sont tels que définis dans la revendication 1, avec un agent réducteur, pour donner un composé de formule XXI, dans laquelle W, X, Z et n sont tels que définis, m est nul, R¹ est un hydrogène et R² est un hydrogène, et
d) la réaction du composé de formule XXI, obtenu dans l'étape c) 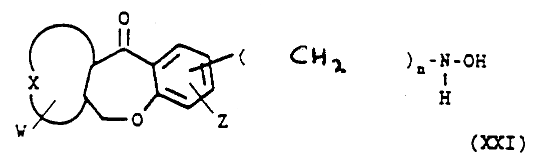 dans laquelle W, X, Z et n sont tels que définis dans la revendication 1, avec un agent d'acylation, pour fournir un composé de formule I, dans laquelle W, X, Z et n sont tels que définis, m est nul et R¹ et R² sont tous deux des groupes acyle.

## Revendications (Revendications pour l'(les) Etat(s) contractant(s) suivant(s): ES, GR)

1. Procédé de préparation d'un composé de formule I 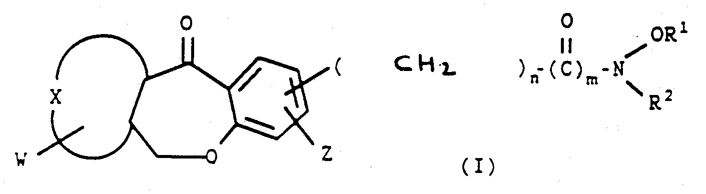 dans laquelle X forme, avec les atomes de carbone auxquels il est fixé, un noyau benzène ou thiophène; W et Z sont, indépendamment, un hydrogène, un halogène, un groupe alkyle en C₁-C₈ ou trifluorométhyle; R¹ est un hydrogène, un groupe alkyle en C₁-C₈ ou acyle; R² est un groupe alkyle en C₁-C₈, cycloalkyle, aryl-alkyle (en C₁-C₈) ou acyle; m a une valeur de 0 ou 1 et n a une valeur de 0 à 12, et de ses sels pharmaceutiquement acceptables, qui comprend
a) la réaction d'un composé de formule II 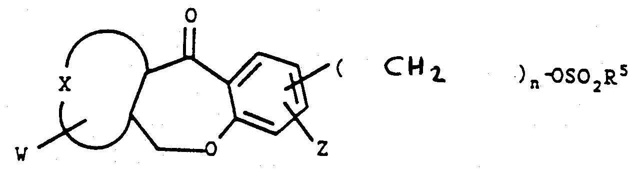 dans laquelle W, X et Z sont tels que définis dans la revendication 1, n est tel que défini ci-dessus, mais n'est pas nul, R⁵ est un groupe alkyle en C₁-C₈, avec un chlorhydrate de N-alkyl(en C₁-C₈)hydroxylamine ou de N-cycloalkylhydroxylamine, pour donner un composé de formule I, dans laquelle W, X, Z et n sont tels que définis ci-dessus, m est nul, R¹ est un hydrogène et R² est un groupe alkyle en C₁-C₈ ou cycloalkyle, ou
b) la réaction d'un composé de formule VII 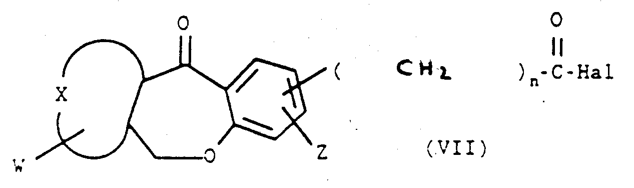 dans laquelle W, X, Z et n sont tels que définis ci-dessus et Hal est un halogène, avec le sel d'une N-alkyl(en C₁-C₈)hydroxylamine ou d'une N-cycloalkylhydroxylamine, pour donner un composé de formule I, dans laquelle W, X, Z et n sont tels que définis ci-dessus, m est égal à 1, R¹ est un hydrogène et R² est un groupe alkyle en C₁-C₈ ou cycloalkyle, ou
c) la réaction d'un composé de formule XX 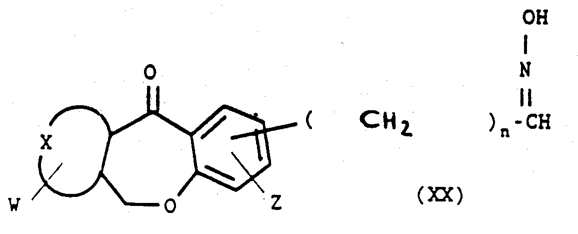 dans laquelle W, X, Z et n sont tels que définis ci-dessus, avec un agent réducteur, pour donner un composé de formule XXI, dans laquelle W, X, Z et n sont tels que définis, m est nul, R¹ est un hydrogène et R² est un hydrogène, et
d) la réaction du composé de formule XXI, obtenu dans l'étape c) 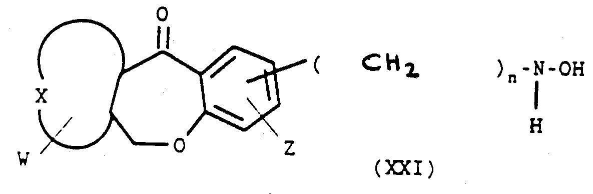 dans laquelle W, X, Z et n sont tels que définis ci-dessus, avec un agent d'acylation, pour fournir un composé de formule I, dans laquelle W, X, Z et n sont tels que définis, m est nul et R¹ et R² sont tous deux des groupes acyle.

2. Procédé tel que défini dans la revendication 1, dans lequel X forme, avec les atomes de carbone auxquels il est fixé, un noyau benzène.

3. Procédé tel que défini dans la revendication 2, dans lequel R¹ est un hydrogène ou un groupe acyle, R² est un groupe alkyle en C₁-C₈, cycloalkyle ou acyle, n a une valeur de 1 à 3 et W et Z sont tous deux des atomes d'hydrogène.

4. Procédé tel que défini dans la revendication 1, dans lequel on prépare la 2-(6,11-dihydroxy-11-oxodibenzo[b,e]oxépine-2-yl)-N-hydroxy-N-méthyl-éthylamine ou un de ses sels pharmaceutiquement acceptables.

5. Procédé tel que défini dans la revendication 1, dans lequel on prépare le 3-(6,11-dihydro-11-oxodibenzo[b,e]oxépine-2-yl)-N-hydroxy-N-méthyl-propanamide, ou un de ses sels pharmaceutiquement acceptables.

6. Procédé tel que défini dans la revendication 1, dans lequel on prépare le N-cyclohexyl-2-(6,11-dihydro-11-oxodibenzo[b,e]oxépine-2-yl)-N-hydroxy-acétamide, ou un de ses sels pharmaceutiquement acceptables.

7. Procédé tel que défini dans la revendication 1, dans lequel on prépare le 2-(6,11-dihydro-11-oxodibenzo[b,e]oxépine-2-yl)-N-éthyl-N-hydroxy-propanamide, ou un de ses sels pharmaceutiquement acceptables.

8. Procédé tel que défini dans la revendication 1, dans lequel on prépare le 3-(6,11-dihydro-11-oxodibenzo[b,e]oxépine-2-yl)-N-cyclohexyl-N-hydroxy-propanamide, ou un de ses sels pharmaceutiquement acceptables.

9. Composé de formule Ia 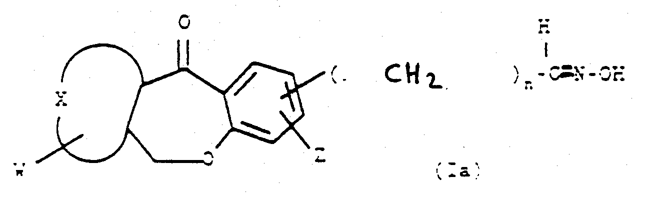 dans laquelle W, X, Z et n sont tels que définis précédemment dans la revendication 1.

10. Utilisation d'un composé tel que défini dans la revendication 1, pour la préparation d'un médicament ayant une activité analgésique ou anti-inflammatoire en topique.
